# EUROPEAN PATENT APPLICATION

(11) **EP 3 825 387 A1**
(43) Date of publication of application: **26.05.2021**
(21) Application number: 20208241.8
(22) Date of filing: 17.11.2020
(51) Int. Cl.: C10L 1/222

(54) **FUEL-SOLUBLE CAVITATION INHIBITOR FOR FUELS USED IN COMMON-RAIL INJECTION ENGINES**

(30) Priority: 22.11.2019 US 201962939039 P
(71) Applicant: Afton Chemical Corporation, Richmond, Virginia 23219 (US)
(72) Inventor: GALANTE-FOX, Julienne M., Midlothian, VA Virginia 23113 (US); LUCADO, Erik, Richmond, VA Virginia 23219 (US)
(74) Representative: SSM Sandmair

(57) **Abstract**

The present disclosure relates to methods and fuel compositions for reducing cavitation-induced damage in common-rail injection engines operated at high fuel pressures. The fuel compositions include a gasoline-like fuel and one or more cavitation inhibitor additives.

## Description

### FIELD

The present disclosure relates to methods for reducing cavitation-induced damage in common-rail injection engines operating at high fuel pressures. More particularly, the disclosure relates to cavitation inhibitors, their use and to methods of reducing cavitation-induced damage in common-rail injection fuel pumps and fuel injectors operating at high fuel pressures by combusting a fuel composition including one or more of the cavitation inhibitor additives.

### BACKGROUND

Heavy-duty engines operating middle distillate fuels (such as diesel and/or jet fuel for example) often present issues with higher than desired emissions and/or particulates due to inherent challenges operating such engines. It would be advantageous if light distillates (such as gasoline for example) could be used in such heavy-duty engine applications in view of the likely reduction in emissions and particulates generated during combustion. Gasoline compression engines or common-rail injection engines, for instance, are one such potential application.

However, gasoline-like fuels (including gasoline) tend to be more volatile and have a much lower viscosity than middle distillate fuels such as diesel. In view of this, the use of gasoline-like fuels in high pressure compression engines tends to increase engine wear due to the differences in properties and the high pressures such engines operate at. For instance, the higher volatility and lower viscosity of gasoline may lead to cavitation of the gasoline-like fuels in the fuel system high pressure pumps and/or fuel injectors. The fuel cavitation may lead to pitting or other damage to these components that may impair the long term operation of such systems.

### BRIEF DESCRIPTION OF THE FIGURES

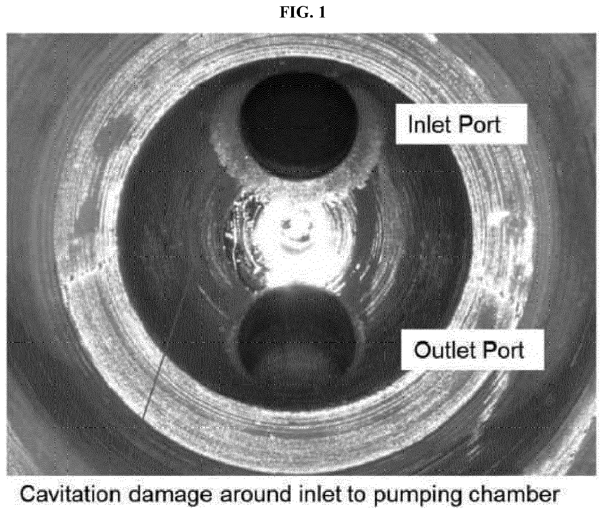

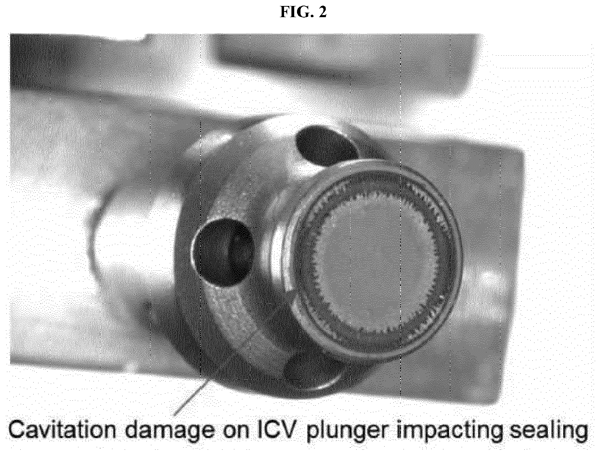

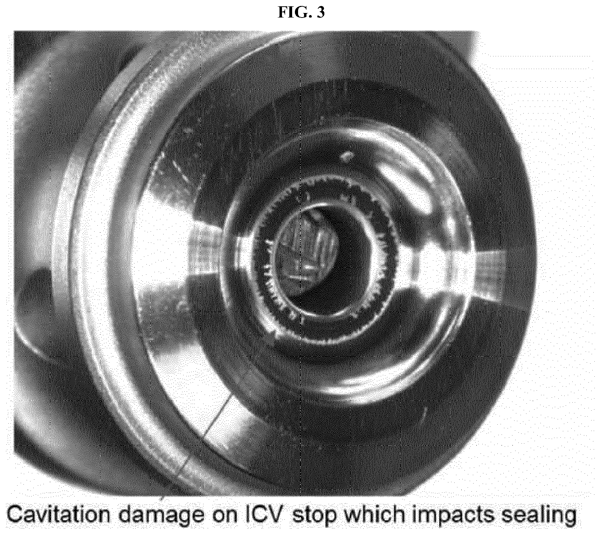

FIGS. 1 to 3 are images of fuel system components.

### SUMMARY

One or more of the foregoing challenges are solved by the use of a cavitation additive including a quaternary ammonium compound in a gasoline-like fuel composition for reducing cavitation damage in a common-rail injection engine, as defined in the appended claims. In one approach or embodiment, a method of reducing cavitation damage in a common-rail injection engine is described by this disclosure. In an aspect, the method includes providing a gasoline-like fuel composition, e.g., gasoline or other light distillate, at a pressure of about 350 to about 3,000 bar to a fuel injector and/or a high pressure pumping system of a common-rail injection engine and combusting the fuel composition in the engine. In other aspect, the fuel composition includes a major amount of gasoline-like fuel, e.g., gasoline or other light distillate, and a minor amount of a cavitation inhibitor including a quaternary ammonium salt.

In some approaches, the quaternary ammonium salt of the previous paragraph is a compound of Formula I: 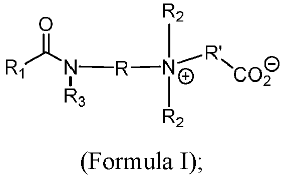 wherein R and R' are independently alkylene linkers having 1 to 10 carbon atoms; R₁ is a hydrocarbyl group or optionally substituted hydrocarbyl group, or an aryl group or optionally substituted aryl group; R₂ is independently a linear or branched C1 to C4 alkyl group; and R₃ is hydrogen or a C1 to C4 alkyl group.

In other approaches or embodiments, the method of reducing cavitation damage in a gasoline engine as described in either of the previous paragraphs may be combined with one or more optional features or method steps. These optional features or steps include any of the following and in any combination: wherein the fuel composition includes about 10 to about 1000 ppmw of the cavitation additive; and/or wherein the engine is a gasoline compression ignition engine and/or a common-rail injection engine; and/or wherein R and R' are independently alkylene linkers having 1 to 3 carbon atoms and R₁ is a C8 to C20 hydrocarbyl group; and/or wherein R' includes a methylene linker; and/or wherein R₂ is a methyl group; and/or wherein the reduction in cavitation damage occurs in one or more of an inlet cavity to a fuel pumping chamber, a fuel inlet check valve plunger, a fuel inlet check valve stop, or any combination thereof.

In yet another approach, the quaternary ammonium salt of the disclosure herein is formed by the reaction of an alkyl carboxylate with an amide or imide compound obtained by reacting a hydrocarbyl substituted acylating agent and an amine, wherein the amine has the structure of Formula II 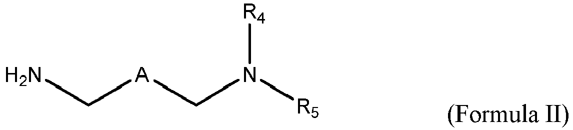 wherein A is a hydrocarbyl linker with 2 to 10 carbon units and including one or more carbon units thereof independently replaced with a bivalent moiety selected from the group consisting of -O-, -N(R')-, -C(O)-, -C(O)O-, -C(O)NR'; R₄ and R₅ are independently alkyl groups containing 1 to 8 carbon atoms; and R' is independently a hydrogen or a group selected from C₁₋₆ aliphatic, phenyl, or alkylphenyl.

The cavitation inhibitor of the preceding paragraph may be combined with one or more optional features either individually or in any combination thereof. These optional features include: wherein the alkyl carboxylate is alkyl oxalate, alkyl salicylate, or a combination thereof; and/or wherein the alkyl group in the alkyl carboxylate is C₁ to C₆ alkyl; and/or wherein A is -(CH₂)ᵣ-[X-(CH₂)_{r'}]ₚ- with each of r, r', and p independently being 1, 2, 3, or 4 and X being oxygen or NR" with R" being hydrogen or a hydrocarbyl group; and/or wherein X is oxygen; and/or wherein the amine is selected from 3-(2-(dimethylamino)ethoxy)propylamine, N,N-dimethyl dipropylene triamine, and mixtures thereof; and/or wherein the hydrocarbyl substitued acylating agent is selected from a hydrocarbyl substituted dicarboxylic acid or anhydride derivative thereof, a fatty acid, or mixtures thereof; and/or wherein the hydrocarbyl substituent has a number average molecular weight of about 200 to about 2500 as measured by GPC using polystyrene as a calibration reference.

### DETAILED DESCRIPTION

The present disclosure describes uses and methods of reducing cavitation-induced damage on fuel system components of a common-rail injection engine operated at high fuel pressures using one or more fuel soluble cavitation inhibitors in gasoline and/or other light distillate fuel. In one approach or embodiment, the fuel soluble cavitation inhibitor includes a quaternary ammonium salt. In some approaches, the quaternary ammonium salt cavitation inhibitors reduce and/or eliminate cavitation-induced damage to fuel system components in a common-rail injection engine when such engines are operated with gasoline and/or other light distillates at high fuel pressures (such as non-idle fuel pressures greater than about 350 bar, greater than 400 bar, greater than 500 bar, greater than about 600 bar, or greater than about 1000 bar, and in other approaches, about 350 to about 5,000 bar, about 500 to about 4,500 bar, about 1,500 to about 3,500 bar, or about 1,500 to about 2,500 bar). It was unexpectedly discovered that the cavitation inhibitors herein reduce or even eliminate cavitation damage in such engines and such fuels.

In one aspect, the cavitation inhibitor is a quaternary ammonium internal salt obtained from amines or polyamines that are substantially devoid of any free anion species. For example, such additive may be made by reacting a tertiary amine of Formula III 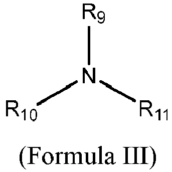 wherein each of R₉, R₁₀, and R₁₁ is selected from hydrocarbyl groups containing from 1 to 200 carbon atoms, with a halogen substituted C2-C8 carboxylic acid, ester, amide, or salt thereof. What is generally to be avoided in the reaction is quaternizing agents selected from the group consisting of hydrocarbyl substituted carboxylates, carbonates, cyclic-carbonates, phenates, epoxides, or mixtures thereof. In one embodiment, the halogen substituted C2-C8 carboxylic acid, ester, amide, or salt thereof may be selected from chloro-, bromo-, fluoro-, and iodo-C2-C8 carboxylic acids, esters, amides, and salts thereof. The salts may be alkali or alkaline earth metal salts selected from sodium, potassium, lithium calcium, and magnesium salts. A particularly useful halogen substituted compound for use in the reaction is the sodium or potassium salt of a chloroacetic acid.

As used herein the term "substantially devoid of free anion species" means that the anions, for the most part are covalently bound to the product such that the reaction product as made does not contain substantial amounts of free anions or anions that are ionically bound to the product. In one embodiment, "substantially devoid" means a range from 0 to less than about 2 wt. % of free anion species, less than about 1.5% wt%, less than about 1 wt%, less than about 0.5 wt%, or none.

In another approach or embodiment, a tertiary amine including monoamines and polyamines may be reacted with the halogen substituted acetic acid, ester, or other derivative thereof to provide cavitation inhibitor additive herein. Suitable tertiary amine compounds are those of Formula IV 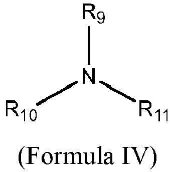 wherein each of R₉, R₁₀, and R₁₁ is selected, as noted above, from hydrocarbyl groups containing from 1 to 200 carbon atoms. Each hydrocarbyl group R₉ to R₁₁ may independently be linear, branched, substituted, cyclic, saturated, unsaturated, or contain one or more hetero atoms. Suitable hydrocarbyl groups may include, but are not limited to alkyl groups, aryl groups, alkylaryl groups, arylalkyl groups, alkoxy groups, aryloxy groups, amido groups, ester groups, imido groups, and the like. Any of the foregoing hydrocarbyl groups may also contain hetero atoms, such as oxygen or nitrogen atoms. Particularly suitable hydrocarbyl groups may be linear or branched alkyl groups. In some approaches, the tertiary amine may be the reaction product of a diamine or triamine with one tertiary amine and a hydrocarbyl substituted carboxylic acid. In other approaches, some representative examples of amine reactants which can be reacted to yield compounds of this disclosure include, but are not limited to, trimethyl amine, triethyl amine, tri-n-propyl amine, dimethylethyl amine, dimethyl lauryl amine, dimethyl oleyl amine, dimethyl stearyl amine, dimethyl eicosyl amine, dimethyl octadecyl amine, N,N-dimethylpropane diamine, N-methyl piperidine, N,N'-dimethyl piperazine, N-methyl-N-ethyl piperazine, N-methyl morpholine, N-ethyl morpholine, N-hydroxyethyl morpholine, pyridine, triethanol amine, triisopropanol amine, methyl diethanol amine, dimethyl ethanol amine, lauryl diisopropanol amine, stearyl diethanol amine, dioleyl ethanol amine, dimethyl isobutanol amine, methyl diisooctanol amine, dimethyl propenyl amine, dimethyl butenyl amine, dimethyl octenyl amine, ethyl didodecenyl amine, dibutyl eicosenyl amine, triethylene diamine, hexamethylenetetramine, N,N,N',N'-tetramethylethylenediamine, N,N,N',N'-tetramethylpropylenediamine, N,N,N',N'-tetraethyl-1,3-propanediamine, methyldi-cyclohexyl amine, 2,6-dimethylpyridine, dimethylcylohexylamine, C10-C30-alkyl or alkenyl-substituted amidopropyldimethylamine, C12-C200-alkyl or alkenyl-substituted succinic-carbonyl-dimethylamine, and the like. A suitable cavitation inhibitor may be the internal salts of oleyl amidopropyl dimethylamino or oleyl dimethyl amine.

If the amine contains solely primary or secondary amino groups, it may be necessary to alkylate at least one of the primary or secondary amino groups to a tertiary amino group prior to the reaction with the halogen substituted C2-C8 carboxylic acid, ester, amide, or salt thereof. In one embodiment, alkylation of primary amines and secondary amines or mixtures with tertiary amines may be exhaustively or partially alkylated to a tertiary amine. It may also be necessary to properly account for the hydrogens on the nitrogen and provide base or acid as required (e.g., alkylation up to the tertiary amine requires removal (neutralization) of the hydrogen (proton) from the product of the alkylation). If alkylating agents, such as, alkyl halides or dialkyl sulfates are used, the product of alkylation of a primary or secondary amine is a protonated salt and needs a source of base to free the amine for further reaction.

The halogen substituted C2-C8 carboxylic acid, ester, amide, or salt thereof for use in making the cavitation inhibitor may be derived from a mono-, di-, or tri- chloro- bromo-, fluoro-, or iodo-carboxylic acid, ester, amide, or salt thereof selected from the group consisting of halogen-substituted acetic acid, propanoic acid, butanoic acid, isopropanoic acid, isobutanoic acid, *tert*-butanoic acid, pentanoic acid, heptanoic acid, octanoic acid, halo-methyl benzoic acid, and isomers, esters, amides, and salts thereof. The salts of the carboxylic acids may include the alkali or alkaline earth metal salts, or ammonium salts including, but not limited to the Na, Li, K, Ca, Mg, triethyl ammonium and triethanol ammonium salts of the halogen-substituted carboxylic acids. A particularly suitable halogen substituted carboxylic acid, ester, or salt thereof may be selected from chloroacetic acid or esters thereof and sodium or potassium chloroacetate. The amount of halogen substituted C2-C8 carboxylic acid, ester, amide, or salt thereof relative to the amount of tertiary amine reactant may range from a molar ratio of about 1:0.1 to about 0.1:1.0.

The internal salts made according to the foregoing procedure may include, but are not limited to (1) hydrocarbyl substituted compounds of the formula R"-NMe₂CH₂COO where R" is from C1 to C30 or a substituted amido group; (2) fatty amide substituted internal salts; and (3) hydrocarbyl substituted imide, amide, or ester internal salts wherein the hydrocarbyl group has 8 to 40 carbon atoms. Particularly suitable internal salts may be selected from the group consisting of polyisobutenyl substituted succinimide, succinic diamide, and succinic diester internal salts; C8-C40 alkenyl substituted succinimide, succinic diamide, and succinic diester internal salts; oleyl amidopropyl dimethylamino internal salts; and oleyl dimethylamino internal salts.

In another aspect, the cavitation inhibitor is the quaternary ammonium salt obtained by reacting (i) the reaction product of a hydrocarbyl-substituted acylating agent and a compound having at least one oxygen or nitrogen atom capable of condensing with said acylating agent and further having a tertiary amino group and (ii) a quaternizing agent.

In component (i), the hydrocarbyl substituted acylating agent is preferably a mono- or polycarboxylic acid (or reactive equivalent thereof) for example a substituted succinic, phthalic or propionic acid.

The hydrocarbyl substituent in such acylating agents preferably comprises at least 8, more preferably at least 12, for example 30 or 50 carbon atoms. It may comprise up to about 200 carbon atoms. Preferably the hydrocarbyl substituent of the acylating agent has a number average molecular weight (Mn) of between 200 to 3000, for example from 250 to 1500, preferably from 500 to 1500 and more preferably 500 to 1100. An Mn of 700 to 1300 is especially preferred, for example from 700 to 1000.

Illustrative of hydrocarbyl substituent based groups containing at least eight carbon atoms are n-octyl, n-decyl, n-dodecyl, tetrapropenyl, n-octadecyl, oleyl, chloroctadecyl, triicontanyl, etc. The hydrocarbyl based substituents may be made from homo- or interpolymers (e.g. copolymers, terpolymers) of mono- and di-olefins having 2 to 10 carbon atoms, for example ethylene, propylene, butane-1, isobutene, butadiene, isoprene, 1-hexene, 1-octene, etc. Preferably these olefins are 1-monoolefins. The hydrocarbyl substituent may also be derived from the halogenated (e.g. chlorinated or brominated) analogs of such homo- or interpolymers. Alternatively the substituent may be made from other sources, for example monomeric high molecular weight alkenes (e.g. 1-tetra-contene) and chlorinated analogs and hydrochlorinated analogs thereof, aliphatic petroleum fractions, for example paraffin waxes and cracked and chlorinated analogs and hydrochlorinated analogs thereof, white oils, synthetic alkenes for example produced by the Ziegler-Natta process (e.g. poly(ethylene) greases) and other sources known to those skilled in the art. Any unsaturation in the substituent may if desired be reduced or eliminated by hydrogenation according to procedures known in the art.

The hydrocarbyl-based substituents are preferably predominantly saturated, that is, they contain no more than one carbon-to-carbon unsaturated bond for every ten carbon to carbon single bonds present. Most preferably they contain no more than one carbon-to-carbon nonaromatic unsaturated bond for every 50 carbon-to-carbon bonds present.

In some preferred embodiments, the hydrocarbyl based substituents are poly-(isobutene)s known in the art. Thus in especially preferred embodiments the hydrocarbyl substituted acylating agent is a polyisobutenyl substituted succinic anhydride.

The preparation of polyisobutenyl substituted succinic anhydrides (PIBSA) is documented in the art. Suitable processes include thermally reacting polyisobutenes with maleic anhydride (see for exampleUS-A-3,361,673 and U.S. Pat. No. 3,018,250), and reacting a halogenated, in particular a chlorinated, polyisobutene (PIB) with maleic anhydride (see for example U.S. Pat. No. 3,172,892). Alternatively, the polyisobutenyl succinic anhydride can be prepared by mixing the polyolefin with maleic anhydride and passing chlorine through the mixture (see for example GB-A-949,981).

Conventional polyisobutenes and so-called "highly reactive" polyisobutenes are suitable for use in the invention. Highly reactive polyisobutenes in this context are defined as polyisobutenes wherein at least 50%, preferably at least 70% or at least 80% or at least 85% or at least 90%, of the terminal olefinic double bonds are of the vinylidene type as described in EP0565285. Particularly preferred polyisobutenes are those having more than 80 mol % and up to 100% of terminal vinylidene groups such as those described in EP1344785.

Other preferred hydrocarbyl groups include those having an internal olefin for example as described in the applicant's published application WO 2007/015080.

An internal olefin as used herein means any olefin containing predominantly a non-alpha double bond, that is a beta or higher olefin. Preferably such materials are substantially completely beta or higher olefins, for example containing less than 10% by weight alpha olefin, more preferably less than 5% by weight or less than 2% by weight. Typical internal olefins include Neodene 151810 available from Shell.

Internal olefins are sometimes known as isomerized olefins and can be prepared from alpha olefins by a process of isomerisation known in the art, or are available from other sources. The fact that they are also known as internal olefins reflects that they do not necessarily have to be prepared by isomerisation.

Examples of the nitrogen or oxygen containing compounds capable of condensing with the acylating agent and further having a tertiary amino group can include but are not limited to: N,N-dimethylaminopropylamine, N,N-diethylaminopropylamine, N,N-dimethylamino ethylamine. The nitrogen or oxygen containing compounds capable of condensing with the acylating agent and further having a tertiary amino group can further include amino alkyl substituted heterocyclic compounds such as l-(3-aminopropyl)imidazole and 4-(3-aminopropyl)morpholine, l-(2-aminoethyl)piperidine, 3,3-diamino-N-methyldipropylamine, and 3'3-aminobis(N,N-dimethylpropylamine). Other types of nitrogen or oxygen containing compounds capable of condensing with the acylating agent and having a tertiary amino group include alkanolamines including but not limited to triethanolamine, trimethanolamine, N,N-dimethylaminopropanol, N,N-dimethylaminoethanol, N,N- diethylaminopropanol, N,N-diethylaminoethanol, N,N-diethylaminobutanol, N,N,N-tris(hydroxyethyl)amine, N,N,N-tris(hydroxymethyl)amine, N,N, N-tris(aminoethyl)amine, N,N-dibutylaminopropylamine and N,N, N'-trimethyl-N'-hydroxyethyl-bisaminoethylether; N,N-bis(3-dimethylaminopropyl)-N-isopropanolamine; N-(3-dimethylaminopropyl)-N,N-diisopropanolamine; N'-(3-(dimethylamino)propyl)-N,N-dimethyl 1,3-propanediamine; 2-(2-dimethylaminoethoxyl)ethanol, N,N,N'-trimethylaminoethylethanolamine, 3-(2-(dimethylamino)ethoxy)propylamine, and N,N-dimethyl dipropylene triamine.

In some preferred embodiments component (i) comprises a compound formed by the reaction of a hydrocarbylsubstituted acylating agent and an amine of formula (V) or (VI): 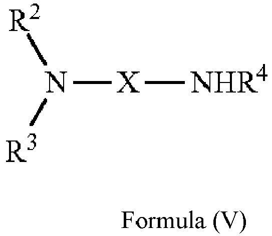 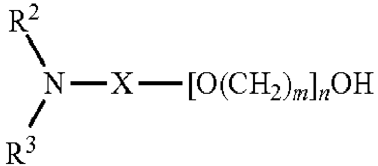 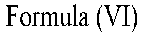 wherein R₂ and R₃ are the same or different alkyl groups having from 1 to 22 carbon atoms; X is an alkylene group having from 1 to 20 carbon atoms that may or may not be interrupted by one or more heteroatoms, such as oxygen or nitrogen; n is from 0 to 20; m is from 1 to 5; and R₄ is hydrogen or a C2 to C22 alkyl group.

When a compound of formula (V) is used, R₄ is preferably hydrogen or a C2 to C16 alkyl group, preferably a C2 to C10 alkyl group, more preferably a C2 to C5 alkyl group. More preferably R₄ is selected from hydrogen, methyl, ethyl, propyl, butyl and isomers thereof. Most preferably R₄ is hydrogen.

When a compound of formula (VI) is used, m is preferably 2 or 3, most preferably 2; n is preferably from 0 to 15, preferably 0 to 10, more preferably from 0 to 5. Most preferably n is 0 and the compound of formula (VI) is an alcohol.

Preferably the hydrocarbyl substituted acylating agent is reacted with a diamine compound of formula (V), wherein X is propylene or propylene-oxo-ethylene.

R₂ and R₃ may each independently be a C2 to C16 alkyl group, preferably a C2 to C10 alkyl group. R₂ and R₃ may independently be methyl, ethyl, propyl, butyl, pentyl, hexyl, heptyl, octyl, or an isomer of any of these. Preferably R₂ and R₃ is each independently C2 to C4 alkyl. Preferably R₂ is methyl. Preferably R₃ is methyl.

X is preferably an alkylene group having 1 to 16 carbon atoms, preferably 1 to 12 carbon atoms, more preferably 1 to 8 carbon atoms, for example 2 to 6 carbon atoms or 2 to 5 carbon atoms. Most preferably X is an ethylene, propylene or butylene group, especially a propylene group.

The preparation of suitable quaternary ammonium salt additives in which the nitrogen-containing species includes component (i) is described in WO 2006/135881.

In preferred embodiments component (i) is the reaction product of a hydrocarbyl-substituted succinic acid derivative (suitably a polyisobutylene-substituted succinic anhydride) and an alcohol or amine also including a tertiary amine group.

In some embodiments when the succinic acid derivative is reacted with an amine (also including a tertiary amine group) under conditions to form a succinimide.

In an alternative embodiment the reaction of the succinic acid derivative and the amine may be carried out.

To form the quaternary ammonium salt additives useful in the present invention, the nitrogen containing species having a tertiary amine group is reacted with a quaternizing agent.

The quatemizing agent is suitably selected from the group consisting of dialkyl sulphates; an ester of a carboxylic acid; alkyl halides; benzyl halides; hydrocarbyl substituted carbonates; and hydrocarbyl epoxides in combination with an acid or mixtures thereof.

In fuel applications it is often desirable to reduce the levels of halogen-, sulfur-, and phosphorus-containing species. Thus if a quatemizing agent containing such an element is used it may be advantageous to carry out a subsequent reaction to exchange the counterion. For example a quarternary ammonium salt formed by reaction with an alkyl halide could be subsequently reacted with sodium hydroxide and the sodium halide salt removed by filtration.

The quatemizing agent can include halides, such as chloride, iodide or bromide; hydroxides; sulphonates; bisulphites, alkyl sulphates, such as dimethyl sulphate; sulphones; phosphates; Cl-12 alkylphosphates; di Cl-12 alkylphosphates; borates; Cl-12 alkylborates; nitrites; nitrates; carbonates; bicarbonates; alkanoates; 0,0-di Cl-12 alkyldithiophosphates; or mixtures thereof.

In one embodiment the quatemizing agent may be derived from dialkyl sulphates such as dimethyl sulphate, N-oxides, sulphones such as propane and butane sulphone; alkyl, acyl or aralkyl halides such as methyl and ethyl chloride, bromide or iodide or benzyl chloride, and a hydrocarbyl (or alkyl) substituted carbonates. If the acyl halide is benzyl chloride, the aromatic ring is optionally further substituted with alkyl or alkenyl groups. The hydrocarbyl (or alkyl) groups of the hydrocarbyl substituted carbonates may contain 1 to 50, 1 to 20, 1 to 10 or 1 to 5 carbon atoms per group. In one embodiment the hydrocarbyl substituted carbonates contain two hydrocarbyl groups that may be the same or different. Examples of suitable hydrocarbyl substituted carbonates include dimethyl or diethyl carbonate.

In another embodiment the quaternizing agent can be a hydrocarbonyl epoxide, as represented by the following Formula (VII): 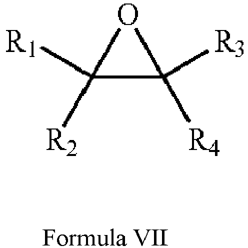 wherein R₁, R₂, R₃ and R₄ can be independently H or a Cl-50 hydrocarbyl group.

Examples of hydrocarbyl epoxides can include styrene oxide, ethylene oxide, propylene oxide, butylene oxide, stilbene oxide and C2-50 epoxide. Styrene oxide is especially preferred.

In some preferred embodiments the quaternizing agent comprises a compound of formula (VIII): 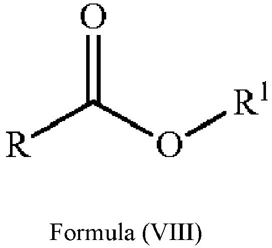 wherein R is an optionally substituted alkyl, alkenyl, aryl or alkylaryl group; and Ri is a to C22 alkyl, aryl or alkylaryl group.

The compound of formula (VIII) is an ester of a carboxylic acid capable of reacting with a tertiary amine to form a quaternary ammonium salt.

Suitable compounds of formula (VIII) include esters of carboxylic acids having a pKa of 3.5 or less. Compound (VIII) may be selected from the diester of oxalic acid, the diester of phthalic acid, the diester of maleic acid, the diester of malonic acid or the diester of citric acid. One especially preferred compound of formula (VIII) is methyl salicylate or dimethyl oxalate.

In yet another approach or embodiment, the cavitation inhibitor may be a quaternary ammonium internal salt of the previously described Formula I 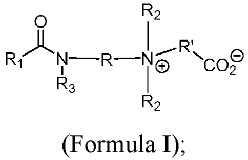 wherein Rand R' are independently alkylene linkers having 1 to 10 carbon atoms (in other approaches 1 to 3 carbon atoms); R₁ is independently a hydrocarbyl group or optionally substituted hydrocarbyl group, or an aryl group or optionally substituted aryl group (in one approach, R₁ is a C8 to C20 hydrocarbyl group); R₂ is independently a linear or branched C1 to C4 alkyl group; R₃ is a hydrogen atom or a C1 to C4 alkyl group. The internal salts of Formula I may also be substantially devoid of free anion species as discussed above.

In another approach, the cavitation inhibitor includes the compound of Formula I above wherein R is a propylene linker, R' is a methylene linker, R₁ is a C8 to C20 hydrocarbyl group, R₂ is a methyl group, and R₃ is hydrogen. In yet other approaches, the cavitation inhibitor is selected from oleyl amidopropyl dimethylamine internal salts or oleyl dimethylamino internal salts. In some approaches, such cavitation inhibitor may be substantially devoid of free anion species.

An exemplary reaction scheme of preparing the cavitation inhibitor suitable for high pressure gasoline engines is shown below in the exemplary multi-step process; of course, other methods of preparing the inhibitors described herein may also be utilized:

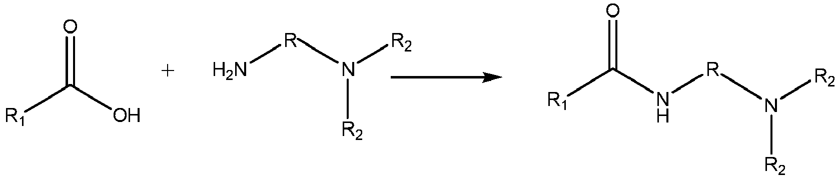

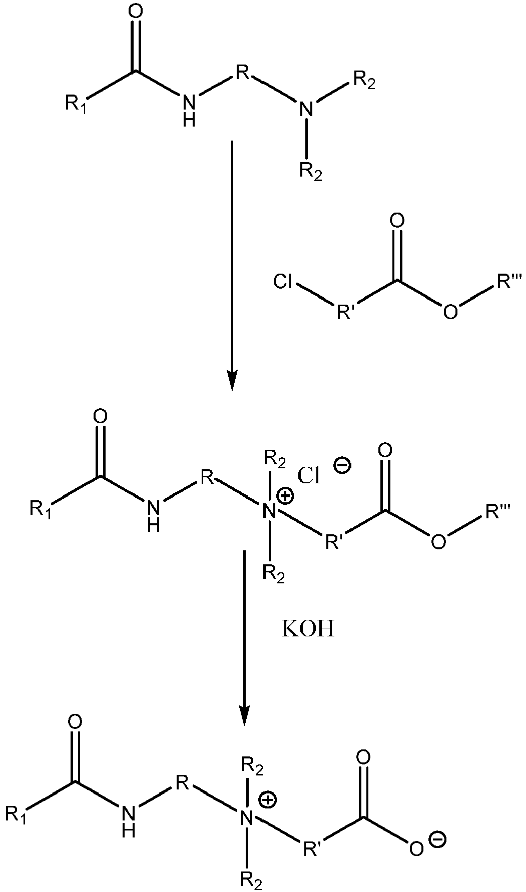

In another approach of this disclosure, an exemplary cavitation inhibitor includes a quaternary ammonium salt formed through a reaction between an alkyl carboxylate and an amide or imide compound obtained by reacting a hydrocarbyl substituted acylating agent and an amine. In one approach of this aspect, the amine has the structure of the previously described Formula II 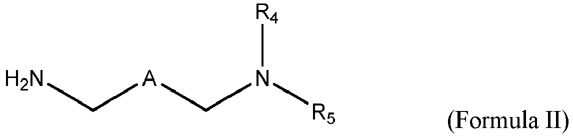 wherein A is a hydrocarbyl linker with 2 to 10 carbon units and including one or more carbon units thereof independently replaced with a bivalent moiety selected from the group consisting of -O-, -N(R')-, -C(O)-, -C(O)O-, and -C(O)NR'. R₄ and R₅ are independently alkyl groups containing 1 to 8 carbon atoms, and R' is independently a hydrogen or a group selected from C₁₋₆ aliphatic, phenyl, or alkylphenyl. In another approach of this aspect, the formed quaternary ammonium salt may be that of Formula II discussed below.

In another aspect of this disclosure, a fuel composition is provided including a major amount of a fuel and a minor amount of a quaternary ammonium salt formed by the reaction of an alkyl carboxylate with an amide or imide compound obtained by reacting a hydrocarbyl substituted acylating agent and an amine, which may be the amine of Formula I above. In one approach of this aspect, the formed quaternary ammonium salt has the structure of Formula IX 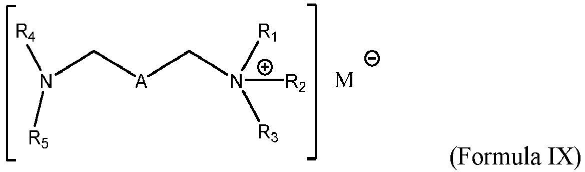 wherein A is a hydrocarbyl linker with 2 to 10 carbon units and including one or more carbon units thereof independently replaced with a bivalent moiety selected from the group consisting of -O-, -N(R')-, -C(O)-, -C(O)O-, and -C(O)NR'. R₁, R₂, and R₃ of Formula V are independently alkyl groups containing 1 to 8 carbon atoms; and R' of Formula V is independently a hydrogen or a group selected from C₁₋₆ aliphatic, phenyl, or alkylphenyl. R₄ and R₅ of Formula V are independently a hydrogen, an acyl group, or a hydrocarbyl substituted acyl group. If one of R₄ or R₅ of Formula v is hydrogen, then the other of R₄ and R₅ is the acyl group or the hydrocarbyl substituted acyl group. If both R₄ and R₅ of Formula v include carbonyl moieties, then one of R₄ and R₅ includes the acyl group and the other of R₄ and R₅ includes the hydrocarbyl substituted acyl group, and R₄ and R₅ together with the N atom to which they are attached, combine to form a ring moiety. In other approaches, R₄ and R₅ together with the N atom to which they are attached, combine to form a hydrocarbyl substituted succinimide. M⁻ is a carboxylate.

As used herein, the term "hydrocarbyl group" or "hydrocarbyl" is used in its ordinary sense, which is well-known to those skilled in the art. Specifically, it refers to a group having a carbon atom directly attached to the remainder of a molecule and having a predominantly hydrocarbon character. Examples of hydrocarbyl groups include: (1) hydrocarbon substituents, that is, aliphatic (e.g., alkyl or alkenyl), alicyclic (e.g., cycloalkyl, cycloalkenyl) substituents, and aromatic-, aliphatic-, and alicyclic-substituted aromatic substituents, as well as cyclic substituents wherein the ring is completed through another portion of the molecule (e.g., two substituents together form an alicyclic radical); (2) substituted hydrocarbon substituents, that is, substituents containing non-hydrocarbon groups which, in the context of the description herein, do not alter the predominantly hydrocarbon substituent (e.g., halo (especially chloro and fluoro), hydroxy, alkoxy, mercapto, alkylmercapto, nitro, nitroso, amino, alkylamino, and sulfoxy); (3) hetero-substituents, that is, substituents which, while having a predominantly hydrocarbon character, in the context of this description, contain other than carbon in a ring or chain otherwise composed of carbon atoms. Hetero-atoms include sulfur, oxygen, nitrogen, and encompass substituents such as pyridyl, furyl, thienyl, and imidazolyl. In general, no more than two, or as a further example, no more than one, non-hydrocarbon substituent will be present for every ten carbon atoms in the hydrocarbyl group; in some embodiments, there will be no non-hydrocarbon substituent in the hydrocarbyl group.

As used herein, the term "major amount" is understood to mean an amount greater than or equal to 50 wt. %, for example from about 80 to about 98 wt .% relative to the total weight of the composition. Moreover, as used herein, the term "minor amount" is understood to mean an amount less than 50 wt. % relative to the total weight of the composition.

In one embodiment, the fuel additives herein are obtained from a select amine having the structure of Formula II 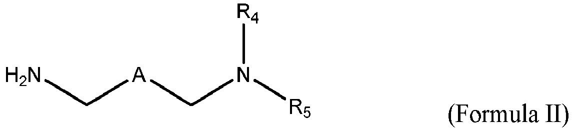 wherein A is a hydrocarbyl linker with 2 to 10 carbon units and including one or more carbon units thereof independently replaced with a bivalent moiety selected from the group consisting of -O-, -N(R')-, -C(O)-, -C(O)O-, and -C(O)NR'. R₄ and R₅ are independently alkyl groups containing 1 to 8 carbon atoms, and R' is independently a hydrogen or a group selected from C₁₋₆ aliphatic, phenyl, or alkylphenyl. In one approach, the select amines of Formula II are at least diamines or triamines having a terminal primary amino group on one end for reaction with the hydrocarbyl substituted acylating agent and a terminal tertiary amine on the other end for reaction with the quaternizing agent. In other approaches, A includes 2 to 6 carbon units with one carbon unit thereof replaced with a -O- or a -NH- group. Suitable exemplary tertiary amine for forming the fuel additives herein may be selected from 3-(2-(dimethylamino)ethoxy)propylamine, N,N-dimethyl dipropylene triamine, and mixtures thereof. In other embodiments or approaches, A has the structure -(CH₂)ᵣ-[X-(CH₂)_{r'}]ₚ- with each of r, r', and p independently being an integer 1, 2, 3, or 4 and X being either oxygen or NR" with R" being hydrogen or a hydrocarbyl group. In other embodiments, X is oxygen. In yet other embodiments, X is -NH-.

The hydrocarbyl linker A preferably has 1 to 4 carbon units replaced with the bivalent moiety described above, which is preferably a -O- or a -NH- group. In other approaches, 1 to 2 carbon units of the hydrocarbyl linker A and, in yet further approaches, 1 carbon unit of the hydrocarbyl linker A is replaced with the bivalent moiety described herein. As appreciated, the remainder of the hydrocarbyl linker A is preferably a carbon atom(s). The number of carbon atoms on either side of the replaced bivalent moiety need not be equal meaning the hydrocarbyl chain between the terminal primary amino group and the terminal tertiary amino group need not be symmetrical relative to the replaced bivalent moiety.

Any of the foregoing described tertiary amines may be reacted with a hydrocarbyl substituted acylating agent that may be selected from a hydrocarbyl substituted mono- di- or polycarboxylic acid or a reactive equivalent thereof to form an amide or imide compound. A particularly suitable acylating agent is a hydrocarbyl substituted succinic acid, ester, anhydride, mono-acid/mono-ester, or diacid. In some approaches, the hydrocarbyl substituted acylating agent is a hydrocarbyl substituted dicarboxylic acid or anhydride derivative thereof, a fatty acid, or mixtures thereof.

In other approaches, the hydrocarbyl substituted acylating agent may be carboxylic acid or anhydride reactant. In one approach, the hydrocarbyl substituted acylating agent may be selected from stearic acid, oleic acid, linoleic acid, linolenic acid, palmitic acid, palmitoleic acid, lauric acid, myristic acid, myristoleic acid, capric acid, caprylic acid, arachidic acid, behenic acid, erucic acid, anhydride derivatives thereof, or a combination thereof.

In one approach, the hydrocarbyl substituted acylating agent is a hydrocarbyl substituted dicarboxylic anhydride of Formula X 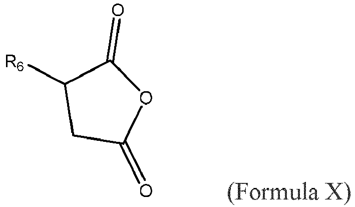 wherein R₆ of Formula IIA is a hydrocarbyl or alkenyl group. In some aspects, R₆ is a hydrocarbyl group having a number average molecular weight from about 200 to about 2500. For example, the number average molecular weight of R₆ may range from about 600 to about 1300, as measured by GPC using polystyrene as a calibration reference. A particularly useful R₆ has a number average molecular weight of about 1000 Daltons and comprises polyisobutylene.

The number average molecular weight (Mn) for any embodiment herein may be determined with a gel permeation chromatography (GPC) instrument obtained from Waters or the like instrument and the data was processed with Waters Empower Software or the like software. The GPC instrument may be equipped with a Waters Separations Module and Waters Refractive Index detector (or the like optional equipment). The GPC operating conditions may include a guard column, 4 Agilent PLgel columns (length of 300×7.5 mm; particle size of 5 µ, and pore size ranging from 100-10000 Å) with the column temperature at about 40 °C. Unstabilized HPLC grade tetrahydrofuran (THF) may be used as solvent, at a flow rate of 1.0 mL/min. The GPC instrument may be calibrated with commercially available polystyrene (PS) standards having a narrow molecular weight distribution ranging from 500 - 380,000 g/mol. The calibration curve can be extrapolated for samples having a mass less than 500 g/mol. Samples and PS standards can be in dissolved in THF and prepared at concentration of 0.1-0.5 wt. % and used without filtration. GPC measurements are also described in US 5,266,223, which is incorporated herein by reference. The GPC method additionally provides molecular weight distribution information; *see, for example,* W. W. Yau, J. J. Kirkland and D. D. Bly, "Modern Size Exclusion Liquid Chromatography", John Wiley and Sons, New York, 1979, also incorporated herein by reference.

In some approaches, the R₆ of formula IIA is a hydrocarbyl moiety that may comprise one or more polymer units chosen from linear or branched alkenyl units. In some aspects, the alkenyl units may have from about 2 to about 10 carbon atoms. For example, the polyalkenyl radical may comprise one or more linear or branched polymer units formed from ethylene radicals, propylene radicals, butylene radicals, pentene radicals, hexene radicals, octene radicals and decene radicals. In some aspects, the R₆ polyalkenyl radical may be in the form of, for example, a homopolymer, copolymer or terpolymer. In other aspects, the polyalkenyl radical is polyisobutylene. For example, the polyalkenyl radical may be a homopolymer of polyisobutylene comprising from about 5 to about 60 isobutylene groups, such as from about 15 to about 30 isobutylene groups. The polyalkenyl compounds used to form the R₆ polyalkenyl radicals may be formed by any suitable methods, such as by conventional catalytic oligomerization of alkenes.

In some aspects, high reactivity polyisobutylenes having relatively high proportions of polymer molecules with a terminal vinylidene group may be used to form the R₆ group. In one example, at least about 60%, such as about 70% to about 90%, of the polyisobutenes comprise terminal olefinic double bonds. In some aspects, approximately one mole of maleic anhydride may be reacted per mole of polyalkylene, such that the resulting polyalkenyl succinic anhydride has about 0.8 to about 1.5 succinic anhydride group per polyalkylene substituent. In other aspects, the molar ratio of succinic anhydride groups to polyalkylene groups may range from about 0.5 to about 3.5, such as from about 1 to about 1.3.

A suitable alkylating or quaternizing agent is a hydrocarbyl-substituted carboxylate, such as an alkyl carboxylate. In some approaches or embodiments, the quaternizing agent is an alkyl carboxylate selected form alkyl oxalate, alkyl salicylate, and combinations thereof. In other approaches or embodiments, the alkyl group of the alkyl carboxylate includes 1 to 6 carbon atoms, and is preferably methyl groups.

For alkylation with an alkyl carboxylate, it may be desirable in some approaches that the corresponding acid of the carboxylate have a pKa of less than 4.2. For example, the corresponding acid of the carboxylate may have a pKa of less than 3.8, such as less than 3.5, with a pKa of less than 3.1 being particularly desirable. Examples of suitable carboxylates may include, but not limited to, maleate, citrate, fumarate, phthalate, 1,2,4-benzenetricarboxylate, 1,2,4,5-benzenetetracarboxylate, nitrobenzoate, nicotinate, oxalate, aminoacetate, and salicylate. As noted above, preferred carboxylates include oxalate, salicylate, and combinations thereof.

In other approaches or embodiments, the quaternary ammonium salt formed by the reaction of an alkyl carboxylate with an amide or imide compound obtained by reacting a hydrocarbyl substituted acylating agent and an amine of Formula II results in a quaternary ammonium salt having the structure of Formula IX 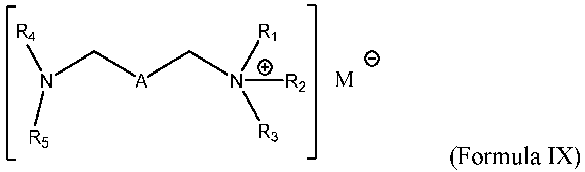 wherein A is a hydrocarbyl linker with 2 to 10 carbon units and including one or more carbon units thereof independently replaced with a bivalent moiety selected from the group consisting of -O-, -N(R')-, -C(O)-, -C(O)O-, or -C(O)NR'. R₁, R₂, and R₃ of Formula V are independently alkyl groups containing 1 to 8 carbon atoms; and R' is independently a hydrogen or a group selected from C₁₋₆ aliphatic, phenyl, or alkylphenyl. R₄ and R₅ of Formula V are independently a hydrogen, an acyl group (RC(O)-), or a hydrocarbyl substituted acyl group (the hydrocarbyl substituted acyl group may be derived from a dicarboxylic acid as shown in the exemplary formulas below). In some approaches or embodiments, if one of R₄ or R₅ is hydrogen, then the other of R₄ and R₅ is the acyl group or the hydrocarbyl substituted acyl group. In other approaches or embodiments, if both R₄ and R₅ include carbonyl moieties, then one of R₄ and R₅ includes the acyl group and the other of R₄ and R₅ includes the hydrocarbyl substitued acyl group, and R₄ and R₅ together with the N atom to which they are attached, combine to form a ring moiety. The hydrocarbyl substituted acyl group may include a terminal carboxyl group. M⁻ is a carboxylate, such as oxalate, salicylate, or combinations thereof.

Suitable examples of the resulting quaternary ammonium salt from the above described reactions include, but are not limited to compounds of the following exemplary structures: 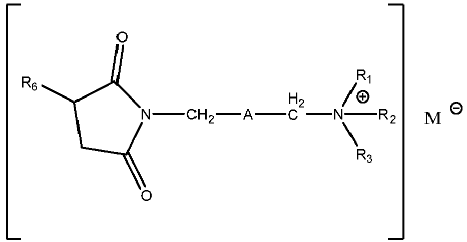 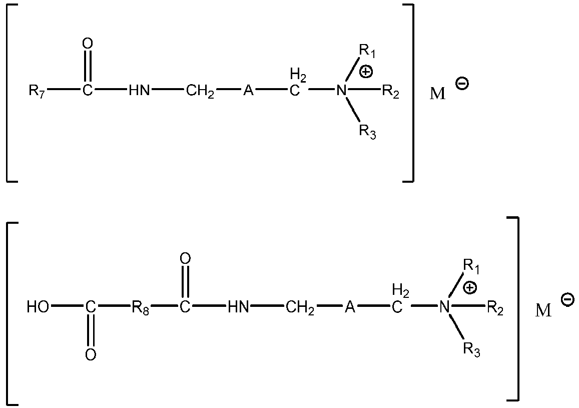 wherein A, R₁, R₂, R₃, R₆, and M are as described above. R₇ is a C1 to C30 hydrocarbyl group, and R₅ is a C1 to C10 hydrocarbyl linker. Due to the length of the hydrocarbyl chain A and the presence of the replacing bivalent moiety therein as discussed above, it is believed the quaternary ammonium salts as described herein include a relatively sterically available quaternary nitrogen that is more available for detergent activity than prior quaternary ammonium compounds.

The cavitation inhibitors herein are particularly useful at reducing and/or eliminating cavitation-induced damage when operating common-rail injection engine with gasoline or other light distillate compositions at high pressures, such as non-idle fuel pressures, greater than 350 bar and, in other approaches, from about 350 to about 3,000 bar (in yet further approaches, greater than about 500 bar and/or from about 1,500 bar to about 2,500 bar). Fuel pressures may be at least about 350 bar, at least about 400 bar, at least about 500 bar, at least about 600 bar, or at least about 1000 bar to about 3,000 bar or less, about 2,500 bar or less, about 2,000 bar or less, or about 1,500 bar or less. Reduction in cavitation damage, generally means the reduction or elimination of cavitation-caused damage largely to fuel system components, such as fuel injectors and high pressure fuel pumps in a gasoline engine when operated at such high pressures. In some approaches, the reduction in cavitation-related damage is a reduction in pitting and other damage to fuel injectors, inlet ports to fuel pumping chambers, plungers for pressure pump inlet check valves, and/or pressure pump inlet check valve stops.

In other approaches or embodiments, the cavitation inhibitor as described in any of the previous paragraphs maybe added to the fuel composition as described herein in amounts up to about 1,000 ppmw (in other approaches up to about 600 ppmw, in yet other approaches, up to about 400 ppmw, up to about 100 ppmw, up to about 50 ppmw, up to about 15 ppmw, and/or up to about 10 ppmw). In other approaches, the cavitation additive is provided to the fuel in amounts of about 5 ppmw to about 1,000 ppmw, in other approaches, about 10 to about 500 ppmw, in yet further approaches, about 40 to about 250 ppmw, and in yet even further approaches about 50 to about 100 ppmw. Other ranges within these endpoints are also possible depending on the circumstances. For instance, the cavitation additives may be provided in gasoline-like fuel in ranges from at least about 5, at least about 10, at least about 20, at least about 30, at least about 40, or at least about 50 ppmw to less than about 1000, less than about 900, less than about 800, less than about 700, less than about 500, less than about 200, less than about 100, less than about 80, less than about 70, or less than about 60 ppmw.

The base fuels used in formulating the fuel compositions of the present disclosure include any base fuels suitable for use in the operation of common-rail injection engines configured to combust fuel at the high fuel pressures discussed herein. Suitable fuels include gasoline fuel compositions, light distillate fuel compositions, and the like and may include leaded or unleaded motor gasolines, and so-called reformulated gasolines which typically contain both hydrocarbons of the gasoline boiling range and fuel-soluble oxygenated blending agents ("oxygenates"), such as alcohols, ethers and other suitable oxygen-containing organic compounds. Preferably, the fuel is a mixture of hydrocarbons boiling in the gasoline boiling range. This fuel may consist of straight chain or branch chain paraffins, cycloparaffins, olefins, aromatic hydrocarbons or any mixture of these. The gasoline can be derived from straight run naptha, polymer gasoline, natural gasoline or from catalytically reformed stocks boiling in the range from about 80° to about 450 °F. The octane level of the gasoline is not critical and any conventional gasoline may be employed in the practice of this invention. The gasoline fuels may have a RON (Research Octane Number) of 50 to 95, a MON (Motor Octane Number) of 55 to 85, and an AKI ((R+N)/2) of 55 to 90.

Oxygenates suitable for use in the present disclosure include methanol, ethanol, isopropanol, t-butanol, mixed C1 to C5 alcohols, methyl tertiary butyl ether, tertiary amyl methyl ether, ethyl tertiary butyl ether and mixed ethers. Oxygenates, when used, will normally be present in the base fuel in an amount below about 30% by volume, and preferably in an amount that provides an oxygen content in the overall fuel in the range of about 0.5 to about 5 percent by volume. Suitable gasoline fuels may have properties as set forth in Table 1 below.

**Table 1: Gasoline-Like Fuel Compositions Configured for the Cavitation Additives**

| **Property** | **Exemplary ASTM** | **Units** | **Possible Exemplary Range** |
|---|---|---|---|
| Initial Boiling Point | D86, ISO 3405 | °C | 30 to 45 |
| 10% Evaporation temperature | D86, ISO 3405 | °C | 50 to 75 |
| 50% Evaporation temperature | D86, ISO 3405 | °C | 80 to 99 |
| 90% evaporation temperature | D86, ISO 3405 | °C | 120 to 155 |
| Final boiling point | D86, ISO 3405 | °C | 134 to 200 |
| Vapor pressure | D5191 | kPa | 45 to 70 |
| Density (15.56 C) | D4052, D1298 | g/ml | 0.71 o 0.73 |
| Kinematic Viscosity | D445 | cSt | 0.55 to 0.59 |
| Wear Scar Diameter | | Um | 200 to 240 |
| Aromatics | D5769, D1319 | Volume % | 7 to 30 |
| Olefins | D6550 | Volume % | 0.7 to 12 |
| Saturates | | Volume % | 60 to 95 |
| Sulfur | D2622, D5453, or D7039 | Ppmw | 3 to 20 |
| H/C ratio | | Mol/mol | 1.5 to 2.5 |
| Cetane Number (CN) | D613, D7170 | - | 35 or below |
| RON | D2699 | - | 50 to 95 |
| MON | D2700 | - | 55 to 85 |
| AKI | (R+M)/2 | - | 55 to 88 |
| Lower Heating value | | MJ/kg | 40 o 45 |

The high pressure common-rail injection engines suitable for the fuels and additives of the present disclosure are engines known to those of ordinary skill that are configured to operate at non-idle fuel pressures greater than about 350 bar and, in other approaches, from about 500 to about 4,500 bar (in yet further approaches, greater than about 500 bar and/or from about 1,500 bar to about 2,500 bar) as previously described. The hydrocarbon or light distillate fuel boiling in the gasoline range or higher than gasoline but lower than diesel may be spark-ignited or compression ignited at such high pressures. Such engines may include individual fuel injectors for each cylinder or combustion chamber of the engine. Suitable engines may include one or more high pressure pumps and suitable high pressure injectors configured to spray fuel into each cylinder or combustion chamber of the engine at the high pressures. In other approaches, the engines may be operated at temperatures effective to combust the gasoline-like fuel under high compression and high pressure. Such engines may be described but are not limited to, for example, in US patent references US 8,235,024; US 8,701,626; US 9,638,146; US 20070250256; and/or US 20060272616 to suggest a few examples. In some instances, the common-rail engine may also be configured to operate at an air-to-fuel weight ratio of about 40:1 or higher in the combustion chamber (in some approaches, about 40:1 to about 70:1 air-to-fuel weight ratio) to deliver fuel at the high pressures noted herein.

Supplemental Fuel Additives: The fuel compositions of the present disclosure may also contain supplemental additives in addition to the cavitation inhibitor described above. For example, supplemental additives may include dispersants, detergents, antioxidants, carrier fluids, metal deactivators, dyes, markers, corrosion inhibitors, biocides, antistatic additives, drag reducing agents, demulsifiers, emulsifiers, dehazers, anti-icing additives, antiknock additives, anti-valve-seat recession additives, lubricity additives, surfactants, combustion improvers, and mixtures thereof.

One particular additional additive may be a Mannich base detergent such as intake valve deposit (IVD) control additive including a Mannich base detergent. Suitable Mannich base detergents for use in the fuel compositions herein include the reaction products of a high molecular weight alkyl-substituted hydroxyaromatic compound, aldehydes and amines. If used, the fuel composition may include about 45 to about 1000 ppm of a Mannich base detergent as a separate IVD control additive.

In one approach, the high molecular weight alkyl substituents on the benzene ring of the hydroxyaromatic compound may be derived from a polyolefin having a number average molecular weight (Mn) from about 500 to about 3000, preferably from about 700 to about 2100, as determined by gel permeation chromatography (GPC) using polystyrene as reference. The polyolefin may also have a polydispersity (weight average molecular weight/number average molecular weight) of about 1 to about 4 (in other instances, about 1 to about 2) as determined by GPC using polystyrene as reference.

The alkylation of the hydroxyaromatic compound is typically performed in the presence of an alkylating catalyst at a temperature in the range of about 0 to about 200 °C, preferably 0 to 100 °C. Acidic catalysts are generally used to promote Friedel-Crafts alkylation. Typical catalysts used in commercial production include sulphuric acid, BF₃, aluminum phenoxide, methanesulphonic acid, cationic exchange resin, acidic clays and modified zeolites.

Polyolefins suitable for forming the high molecular weight alkyl-substituted hydroxyaromatic compounds include polypropylene, polybutenes, polyisobutylene, copolymers of butylene and/or butylene and propylene, copolymers of butylene and/or isobutylene and/or propylene, and one or more mono-olefinic comonomers copolymerizable therewith (e.g., ethylene, 1-pentene, 1-hexene, 1-octene, 1-decene, etc.) where the copolymer molecule contains at least 50% by weight, of butylene and/or isobutylene and/or propylene units. The comonomers polymerized with propylene or such butenes may be aliphatic and can also contain non-aliphatic groups, e.g., styrene, o-methylstyrene, p-methylstyrene, divinyl benzene and the like. Thus in any case the resulting polymers and copolymers used in forming the high molecular weight alkyl-substituted hydroxyaromatic compounds are substantially aliphatic hydrocarbon polymers.

Polybutylene is preferred. Unless otherwise specified herein, the term "polybutylene" is used in a generic sense to include polymers made from "pure" or "substantially pure" 1-butene or isobutene, and polymers made from mixtures of two or all three of 1-butene, 2-butene and isobutene. Commercial grades of such polymers may also contain insignificant amounts of other olefins. So-called high reactivity polyisobutenes having relatively high proportions of polymer molecules having a terminal vinylidene group are also suitable for use in forming the long chain alkylated phenol reactant. Suitable high-reactivity polyisobutenes include those polyisobutenes that comprise at least about 20% of the more reactive methylvinylidene isomer, preferably at least 50% and more preferably at least 70%. Suitable polyisobutenes include those prepared using BF₃ catalysts. The preparation of such polyisobutenes in which the methylvinylidene isomer comprises a high percentage of the total composition is described in US 4,152,499 and US 4,605,808, which are both incorporated herein by reference.

The Mannich detergent may be made from a high molecular weight alkylphenol or alkylcresol. However, other phenolic compounds may be used including high molecular weight alkyl-substituted derivatives of resorcinol, hydroquinone, catechol, hydroxydiphenyl, benzylphenol, phenethylphenol, naphthol, tolylnaphthol, among others. Preferred for the preparation of the Mannich detergents are the polyalkylphenol and polyalkylcresol reactants, e.g., polypropylphenol, polybutylphenol, polypropylcresol and polybutylcresol, wherein the alkyl group has a number average molecular weight of about 500 to about 2100 as measured by GPC using polystyrene as reference, while the most preferred alkyl group is a polybutyl group derived from polyisobutylene having a number average molecular weight in the range of about 700 to about 1300 as measured by GPC using polystyrene as reference.

The preferred configuration of the high molecular weight alkyl-substituted hydroxyaromatic compound is that of a para-substituted mono-alkylphenol or a para-substituted mono-alkyl ortho-cresol. However, any hydroxyaromatic compound readily reactive in the Mannich condensation reaction may be employed. Thus, Mannich products made from hydroxyaromatic compounds having only one ring alkyl substituent, or two or more ring alkyl substituents are suitable for use in this invention. The long chain alkyl substituents may contain some residual unsaturation, but in general, are substantially saturated alkyl groups.

Representative amine reactants include, but are not limited to, alkylene polyamines having at least one suitably reactive primary or secondary amino group in the molecule. Other substituents such as hydroxyl, cyano, amido, etc., can be present in the polyamine. In a preferred embodiment, the alkylene polyamine is a polyethylene polyamine. Suitable alkylene polyamine reactants include ethylenediamine, diethylenetriamine, triethylenetetramine, tetraethylenepentamine and mixtures of such amines having nitrogen contents corresponding to alkylene polyamines of the formula H₂N--(A-NH--)ₙH, where A in this formula is divalent ethylene or propylene and n is an integer of from 1 to 10, preferably 1 to 4. The alkylene polyamines may be obtained by the reaction of ammonia and dihalo alkanes, such as dichloro alkanes.

The amine may also be an aliphatic diamine having one primary or secondary amino group and at least one tertiary amino group in the molecule. Examples of suitable polyamines include N,N,N",N"-tetraalkyldialkylenetriamines (two terminal tertiary amino groups and one central secondary amino group), N,N,N',N"-tetraalkyltrialkylenetetramines (one terminal tertiary amino group, two internal tertiary amino groups and one terminal primary amino group), N,N,N',N",N"'-pentaalkyltrialkylenetetramines (one terminal tertiary amino group, two internal tertiary amino groups and one terminal secondary amino group), N,N-dihydroxyalkyl-alpha-, omega-alkylenediamines (one terminal tertiary amino group and one terminal primary amino group), N,N,N'-trihydroxyalkyl-alpha, omega-alkylenediamines (one terminal tertiary amino group and one terminal secondary amino group), tris(dialkylaminoalkyl)aminoalkylmethanes (three terminal tertiary amino groups and one terminal primary amino group), and similar compounds, wherein the alkyl groups are the same or different and typically contain no more than about 12 carbon atoms each, and which preferably contain from 1 to 4 carbon atoms each. Most preferably these alkyl groups are methyl and/or ethyl groups. Preferred polyamine reactants are N,N-dialkyl-alpha, omega-alkylenediamine, such as those having from 3 to about 6 carbon atoms in the alkylene group and from 1 to about 12 carbon atoms in each of the alkyl groups, which most preferably are the same but which can be different. Most preferred is N,N-dimethyl-1,3-propanediamine and N-methyl piperazine.

Examples of polyamines having one reactive primary or secondary amino group that can participate in the Mannich condensation reaction, and at least one sterically hindered amino group that cannot participate directly in the Mannich condensation reaction to any appreciable extent include N-(*tert*-butyl)-1,3-propanediamine, N-neopentyl-1,3-propanediamine- , *N-*(*tert-*butyl)-1-methyl-1,2-ethanediamine, N-(tert-butyl)-1-methyl-1,3-p- ropanediamine, and 3,5-di(*tert*-butyl)aminoethylpiperazine.

Representative aldehydes for use in the preparation of the Mannich base products include the aliphatic aldehydes such as formaldehyde, acetaldehyde, propionaldehyde, butyraldehyde, valeraldehyde, caproaldehyde, heptaldehyde, stearaldehyde. Aromatic aldehydes which may be used include benzaldehyde and salicylaldehyde. Illustrative heterocyclic aldehydes for use herein are furfural and thiophene aldehyde, etc. Also useful are formaldehyde-producing reagents such as paraformaldehyde, or aqueous formaldehyde solutions such as formalin. Most preferred is formaldehyde or formalin.

The condensation reaction among the alkylphenol, the specified amine(s) and the aldehyde may be conducted at a temperature typically in the range of about 40 °C to about 200 °C. The reaction can be conducted in bulk (no diluent or solvent) or in a solvent or diluent. Water is evolved and can be removed by azeotropic distillation during the course of the reaction. Typically, the Mannich reaction products are formed by reacting the alkyl-substituted hydroxyaromatic compound, the amine and aldehyde in the molar ratio of 1.0:0.5-2.0:1.0-3.0, respectively.

Suitable Mannich base detergents include those detergents taught in US 4,231,759; US 5,514,190; US 5,634,951; US 5,697,988; US 5,725,612; and 5,876,468, the disclosures of which are incorporated herein by reference.

Another suitable additional fuel additive may be a hydrocarbyl amine detergent. If used, the fuel composition may include about 45 to about 1000 ppm of the hydrocarbyl amine detergent. One common process involves halogenation of a long chain aliphatic hydrocarbon such as a polymer of ethylene, propylene, butylene, isobutene, or copolymers such as ethylene and propylene, butylene and isobutylene, and the like, followed by reaction of the resultant halogenated hydrocarbon with a polyamine. If desired, at least some of the product can be converted into an amine salt by treatment with an appropriate quantity of an acid. The products formed by the halogenation route often contain a small amount of residual halogen such as chlorine. Another way of producing suitable aliphatic polyamines involves controlled oxidation (e.g., with air or a peroxide) of a polyolefin such as polyisobutene followed by reaction of the oxidized polyolefin with a polyamine. For synthesis details for preparing such aliphatic polyamine detergent/dispersants, see for example U.S. Pat. Nos. 3,438,757; 3,454,555; 3,485,601; 3,565,804; 3,573,010; 3,574,576; 3,671,511; 3,746,520; 3,756,793; 3,844,958; 3,852,258; 3,864,098; 3,876,704; 3,884,647; 3,898,056; 3,950,426; 3,960,515; 4,022,589; 4,039,300; 4,128,403; 4,166,726; 4,168,242; 5,034,471; 5,086,115; 5,112,364; and 5,124,484; and published European Patent Application 384,086. The disclosures of each of the foregoing documents are incorporated herein by reference. The long chain substituent(s) of the hydrocarbyl amine detergent most preferably contain(s) an average of 40 to 350 carbon atoms in the form of alkyl or alkenyl groups (with or without a small residual amount of halogen substitution). Alkenyl substituents derived from poly-alpha-olefin homopolymers or copolymers of appropriate molecular weight (e.g., propene homopolymers, butene homopolymers, C3 and C4 alpha-olefin copolymers, and the like) are suitable. Most preferably, the substituent is a polyisobutenyl group formed from polyisobutene having a number average molecular weight (as determined by gel permeation chromatography) in the range of 500 to 2000, preferably 600 to 1800, most preferably 700 to 1600.

Polyetheramines are yet another suitable additional detergent chemistry used in the methods of the present disclosure. If used, the fuel composition may include about 45 to about 1000 ppm of the polyetheramine detergents. The poly ether backbone in such detergents can be based on propylene oxide, ethylene oxide, butylene oxide, or mixtures of these. The most preferred are propylene oxide or butylene oxide or mixture thereof to impart good fuel solubility. The polyetheramines can be monoamines, diamines or triamines. Examples of commercially available polyetheramines are those under the tradename Jeffamines™ available from Huntsman Chemical Company and the poly(oxyalkylene)carbamates available from Chevron Chemical Company. The molecular weight of the polyetheramines will typically range from 500 to 3000. Other suitable polyetheramines are those compounds taught in U.S. Pat. Nos. 4,191,537; 4,236,020; 4,288,612; 5,089,029; 5,112,364; 5,322,529; 5,514,190 and 5,522,906.

In some approaches, the fuel-soluble synergistic detergent mixture may also be used with a liquid carrier or induction aid. Such carriers can be of various types, such as for example liquid poly-α-olefin oligomers, mineral oils, liquid poly(oxyalkylene) compounds, liquid alcohols or polyols, polyalkenes, liquid esters, and similar liquid carriers. Mixtures of two or more such carriers can be employed.

Exemplary liquid carriers may include a mineral oil or a blend of mineral oils that have a viscosity index of less than about 120; one or more poly-α-olefin oligomers; one or more poly(oxyalkylene) compounds having an average molecular weight in the range of about 500 to about 3000; polyalkenes; polyalkyl-substituted hydroxyaromatic compounds; or mixtures thereof. The mineral oil carrier fluids that can be used include paraffinic, naphthenic and asphaltic oils, and can be derived from various petroleum crude oils and processed in any suitable manner. For example, the mineral oils may be solvent extracted or hydrotreated oils. Reclaimed mineral oils can also be used. Hydrotreated oils are the most preferred. Preferably the mineral oil used has a viscosity at 40 °C of less than about 1600 SUS, and more preferably between about 300 and 1500 SUS at 40 °C. Paraffinic mineral oils most preferably have viscosities at 40 °C in the range of about 475 SUS to about 700 SUS. In some instances, the mineral oil may have a viscosity index of less than about 100, in other instances, less than about 70 and, in yet further instances, in the range of from about 30 to about 60.

The poly-α-olefins (PAO) suitable for use as carrier fluids are the hydrotreated and unhydrotreated poly-α-olefin oligomers, such as, hydrogenated or unhydrogenated products, primarily trimers, tetramers and pentamers of alpha-olefin monomers, which monomers contain from 6 to 12, generally 8 to 12 and most preferably about 10 carbon atoms. Their synthesis is outlined in Hydrocarbon Processing, February 1982, page 75 et seq., and in U.S. Pat. Nos. 3,763,244; 3,780,128; 4,172,855; 4,218,330; and 4,950,822. The usual process essentially comprises catalytic oligomerization of short chain linear alpha olefins (suitably obtained by catalytic treatment of ethylene). The poly-α-olefins used as carriers will usually have a viscosity (measured at 100 °C) in the range of 2 to 20 centistokes (cSt). Preferably, the poly-α-olefin has a viscosity of at least 8 cSt, and most preferably about 10 cSt at 100 °C.

Suitable poly (oxyalkylene) compounds for the carrier fluids may be fuel-soluble compounds which can be represented by the following formula

R_{A}--(R_{B}-O)_{w}--R_{C}

wherein R_{A} is typically a hydrogen, alkoxy, cycloalkoxy, hydroxy, amino, hydrocarbyl (e.g., alkyl, cycloalkyl, aryl, alkylaryl, aralkyl, etc.), amino-substituted hydrocarbyl, or hydroxy-substituted hydrocarbyl group, R_{B} is an alkylene group having 2 to 10 carbon atoms (preferably 2-4 carbon atoms), R_{C} is typically a hydrogen, alkoxy, cycloalkoxy, hydroxy, amino, hydrocarbyl (e.g., alkyl, cycloalkyl, aryl, alkylaryl, aralkyl, etc.), amino-substituted hydrocarbyl, or hydroxy-substituted hydrocarbyl group, and w is an integer from 1 to 500 and preferably in the range of from 3 to 120 representing the number (usually an average number) of repeating alkyleneoxy groups. In compounds having multiple --R_{B}--O-- groups, R_{B} can be the same or different alkylene group and where different, can be arranged randomly or in blocks. Preferred poly (oxyalkylene) compounds are monools comprised of repeating units formed by reacting an alcohol with one or more alkylene oxides, preferably one alkylene oxide, more preferably propylene oxide or butylene oxide.

The average molecular weight of the poly (oxyalkylene) compounds used as carrier fluids is preferably in the range of from about 500 to about 3000, more preferably from about 750 to about 2500, and most preferably from above about 1000 to about 2000.

One useful sub-group of poly (oxyalkylene) compounds is comprised of the hydrocarbyl-terminated poly(oxyalkylene) monools such as are referred to in the passage at column 6, line 20 to column 7 line 14 of U.S. Pat. No. 4,877,416 and references cited in that passage, said passage and said references being fully incorporated herein by reference.

Another sub-group of poly (oxyalkylene) compounds includes one or a mixture of alkylpoly (oxyalkylene)monools which in its undiluted state is a gasoline-soluble liquid having a viscosity of at least about 70 centistokes (cSt) at 40 °C and at least about 13 cSt at 100 °C. Of these compounds, monools formed by propoxylation of one or a mixture of alkanols having at least about 8 carbon atoms, and more preferably in the range of about 10 to about 18 carbon atoms, are particularly preferred.

The poly (oxyalkylene) carriers may have viscosities in their undiluted state of at least about 60 cSt at 40 °C (in other approaches, at least about 70 cSt at 40 °C) and at least about 11 cSt at 100 °C (more preferably at least about 13 cSt at 100 °C). In addition, the poly (oxyalkylene) compounds used in the practice of this invention preferably have viscosities in their undiluted state of no more than about 400 cSt at 40 °C and no more than about 50 cSt at 100 °C. In other approaches, their viscosities typically do not exceed about 300 cSt at 40 °C and typically do not exceed about 40 cSt at 100 °C.

Preferred poly (oxyalkylene) compounds also include poly (oxyalkylene) glycol compounds and monoether derivatives thereof that satisfy the above viscosity requirements and that are comprised of repeating units formed by reacting an alcohol or polyalcohol with an alkylene oxide, such as propylene oxide and/or butylene oxide with or without use of ethylene oxide, and especially products in which at least 80 mole % of the oxyalkylene groups in the molecule are derived from 1,2-propylene oxide. Details concerning preparation of such poly(oxyalkylene) compounds are referred to, for example, in Kirk-Othmer, Encyclopedia of Chemical Technology, Third Edition, Volume 18, pages 633-645 (Copyright 1982 by John Wiley & Sons), and in references cited therein, the foregoing excerpt of the Kirk-Othmer encyclopedia and the references cited therein being incorporated herein by reference. U.S. Pat. Nos. 2,425,755; 2,425,845; 2,448,664; and 2,457,139 also describe such procedures, and are fully incorporated herein by reference.

The poly (oxyalkylene) compounds, when used, typically will contain a sufficient number of branched oxyalkylene units (e.g., methyldimethyleneoxy units and/or ethyldimethyleneoxy units) to render the poly (oxyalkylene) compound gasoline soluble. Suitable poly (oxyalkylene) compounds include those taught in U.S. Pat. Nos. 5,514,190; 5,634,951; 5,697,988; 5,725,612; 5,814,111 and 5,873,917, the disclosures of which are incorporated herein by reference.

The polyalkenes suitable for use as carrier fluids include polypropene and polybutene. The polyalkenes may have a polydispersity (Mw/Mn) of less than 4. In one embodiment, the polyalkenes have a polydispersity of 1.4 or below. In general, polybutenes have a number average molecular weight (Mn) of about 500 to about 2000, preferably 600 to about 1000, as determined by gel permeation chromatography (GPC). Suitable polyalkenes for use in the present invention are taught in U.S. Pat. No. 6,048,373.

The polyalkyl-substituted hydroxyaromatic compounds suitable for use as carrier fluid include those compounds known in the art as taught in U.S. Pat. Nos. 3,849,085; 4,231,759; 4,238,628; 5,300,701; 5,755,835 and 5,873,917, the disclosures of which are incorporated herein by reference.

### DEFINITIONS

For purposes of this disclosure, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausolito: 1999, and "March's Advanced Organic Chemistry", 5th Ed., Ed.: Smith, M.B. and March, J., John Wiley & Sons, New York: 2001, the entire contents of which are hereby incorporated by reference.

As used herein, the term "major amount" is understood to mean an amount greater than or equal to 50 wt. %, for example from about 80 to about 98 wt. % relative to the total weight of the composition. Moreover, as used herein, the term "minor amount" is understood to mean an amount less than 50 wt. % relative to the total weight of the composition.

As described herein, compounds may optionally be substituted with one or more substituents, such as are illustrated generally above, or as exemplified by particular classes, subclasses, and species of the disclosure.

As used herein, an "alkyl" group refers to a saturated aliphatic hydrocarbon group containing (unless otherwise noted in this disclosure) 1-12 (e.g., 1-8, 1-6, or 1-4) carbon atoms. An alkyl group can be straight or branched. Examples of alkyl groups include, but are not limited to, methyl, ethyl, propyl, isopropyl, butyl, isobutyl, sec-butyl, *tert-butyl, n*-pentyl, *n-*heptyl, or 2-ethylhexyl. An alkyl group can be substituted (i.e., optionally substituted) with one or more substituents such as halo, phospho, cycloaliphatic [e.g., cycloalkyl or cycloalkenyl], heterocycloaliphatic [e.g., heterocycloalkyl or hetero cycloalkenyl], aryl, heteroaryl, alkoxy, aroyl, heteroaroyl, acyl [e.g., (aliphatic)carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (heterocyclo alkyl)carbonylamino, (heterocycloalkylalkyl) carbonylamino, heteroarylcarbonylamino, heteroaralkylcarbonylamino alkylaminocarbonyl, cycloalkylaminocarbonyl, heterocyclo alkylaminocarbonyl, arylaminocarbonyl, or heteroarylaminocarbonyl], amino [e.g., aliphaticamino, cycloaliphaticamino, or heterocycloaliphaticamino], sulfonyl [e.g., aliphatic-SO₂-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroarylalkoxy, alkoxycarbonyl, alkylcarbonyloxy, or hydroxy. Without limitation, some examples of substituted alkyls include carboxyalkyl (such as HOOC-alkyl, alkoxy carbonylalkyl, and alkylcarbonyloxyalkyl), cyanoalkyl, hydroxyalkyl, alkoxyalkyl, acylalkyl, aralkyl, (alkoxyaryl)alkyl, (sulfonylamino)alkyl (such as (alkyl-SO₂-amino)alkyl), aminoalkyl, amidoalkyl, (cycloaliphatic)alkyl, or haloalkyl.

As used herein, an "alkenyl" group refers to an aliphatic carbon group that contains (unless otherwise noted in this disclosure) 2-8 (e.g., 2-12, 2-6, or 2-4) carbon atoms and at least one double bond. Like an alkyl group, an alkenyl group can be straight or branched. Examples of an alkenyl group include, but are not limited to allyl, isoprenyl, 2-butenyl, and 2-hexenyl. An alkenyl group can be optionally substituted with one or more substituents such as halo, phospho, cycloaliphatic [e.g., cycloalkyl or cycloalkenyl], heterocycloaliphatic [e.g., heterocycloalkyl or hetero cycloalkenyl], aryl, heteroaryl, alkoxy, aroyl, heteroaroyl, acyl [e.g., (aliphatic) carbonyl, (cycloaliphatic)carbonyl, or (heterocycloaliphatic)carbonyl], nitro, cyano, amido [e.g., (cycloalkylalkyl)carbonylamino, arylcarbonylamino, aralkylcarbonylamino, (hetero cycloalkyl) carbonylamino, (heterocyclo alkylalkyl) carbonylamino, heteroarylcarbonylamino, heteroaralkylcarbonylamino alkylaminocarbonyl, cycloalkylaminocarbonyl, hetero cyclo alkylaminocarbonyl, arylaminocarbonyl, or heteroarylaminocarbonyl], amino [e.g., aliphaticamino, cycloaliphaticamino, heterocyclo aliphaticamino, or aliphaticsulfonylamino], sulfonyl [e.g., alkyl-SO₂-, cycloaliphatic-SO₂-, or aryl-SO₂-], sulfinyl, sulfanyl, sulfoxy, urea, thiourea, sulfamoyl, sulfamide, oxo, carboxy, carbamoyl, cycloaliphaticoxy, heterocycloaliphaticoxy, aryloxy, heteroaryloxy, aralkyloxy, heteroaralkoxy, alkoxycarbonyl, alkylcarbonyloxy, or hydroxy. Without limitation, some examples of substituted alkenyls include cyanoalkenyl, alkoxyalkenyl, acylalkenyl, hydroxyalkenyl, aralkenyl, (alkoxyaryl)alkenyl, (sulfonylamino)alkenyl (such as (alkyl-SO₂-amino)alkenyl), aminoalkenyl, amidoalkenyl, (cycloaliphatic)alkenyl, or haloalkenyl.

A hydrocarbyl group refers to a group that has a carbon atom directly attached to a remainder of the molecule and each hydrocarbyl group is independently selected from hydrocarbon substituents, and substituted hydrocarbon substituents may contain one or more of halo groups, hydroxyl groups, alkoxy groups, mercapto groups, nitro groups, nitroso groups, amino groups, sulfoxy groups, pyridyl groups, furyl groups, thienyl groups, imidazolyl groups, sulfur, oxygen and nitrogen, and wherein no more than two non-hydrocarbon substituents are present for every ten carbon atoms in the hydrocarbyl group.

As used herein, gasoline-like fuel means a liquid distillate obtained from oil refinery which is more volatile and have much lower viscosity than diesel. In some embodiments, gasoline-like fuel has a boiling range between 30-300°C or 30-280°C or 30-250°C or 30-210°C or 40-175°C or 40-150°C or 40-100°C or 100-300°C or 150-275°C. In some other embodiments, the gasoline-like fuel has a final boiling point lower than 275°C, lower than 210°C, lower than 200°C, lower than 180°C, or lower than 150°C. In any of the above-mentioned embodiments, the gasoline-like fuel may also have a kinematic viscosity at 40°C lower than 2.2 cSt or lower than 2.0 cSt or lower than 1.8 cSt or lower than 1.5 cSt or lower than 1.0 cSt or lower than 0.6 cSt. The gasoline-like fuel includes gasoline, an example of which is RON60 gasoline shown in Table 2 below. In addition, the gasoline-like fuel may have a cetane number between 25-46, between 30-42, between 32-40, or between 34-38; and/or have a vapor pressure between 7-70 kPa, between 10-65 kPa, between 20-60 kPa, between 30-50 kPa, or between 40-50 kPa. The gasoline-like fuel may also comprise 0.1-80% biofuel, such as methanol or ethanol.

As used herein, fuel-soluble generally means that the substance should be sufficiently soluble (or dissolve) at about 20 °C in the gasoline-like fuel at least at the minimum concentration required for the substance to serve its intended function. Preferably, the substance will have a substantially greater solubility in the base fuel. However, the substance need not dissolve in the base fuel in all proportions.

The number average molecular weight (Mn) for any approach, aspect, embodiment or Example herein may be determined with a gel permeation chromatography (GPC) instrument obtained from Waters or the like instrument and data as processed with Waters Empower Software or the like software. The GPC instrument may be equipped with a Waters Separations Module and Waters Refractive Index detector (or the like optional equipment). The GPC operating conditions may include a guard column, 4 Agilent PLgel columns (length of 300×7.5 mm; particle size of 5 µ, and pore size ranging from 100-10000 Å) with the column temperature at about 40 °C. Unstabilized HPLC grade tetrahydrofuran (THF) may be used as solvent, at a flow rate of 1.0 mL/min. The GPC instrument may be calibrated with commercially available polystyrene (PS) standards having a narrow molecular weight distribution ranging from 500 - 380,000 g/mol. The calibration curve can be extrapolated for samples having a mass less than 500 g/mol. Samples and PS standards can be in dissolved in THF and prepared at concentration of 0.1-0.5 wt. % and used without filtration. GPC measurements are also described in US 5,266,223, which is incorporated herein by reference. The GPC method additionally provides molecular weight distribution information; *see, for example,* W. W. Yau, J. J. Kirkland and D. D. Bly, "Modern Size Exclusion Liquid Chromatography", John Wiley and Sons, New York, 1979, also incorporated herein by reference.

A better understanding of the present disclosure and its many advantages may be clarified with the following examples. The following examples are illustrative and not limiting thereof in either scope or spirit. Those skilled in the art will readily understand that variations of the components, methods, steps, and devices described in these examples can be used. Unless noted otherwise or apparent from the context of discussion, all percentages, ratios, and parts noted in this disclosure are by weight.

### EXAMPLES

### EXAMPLE 1

A mixture of oleyl amidopropyl dimethylamine (OD, 366 grams) and sodium chloroacetate (SCA, 113 grams) was heated in a mixture of isopropanol (125 mL) and water (51 grams) at 80° C. for 5.5 hours. Isopropanol (600 mL) and 2-ethylhexanol (125 grams) were added and the mixture was concentrated by heating to remove water. The resultant mixture was filtered through CELITE 512 filter medium to give product as a yellow oil.

### EXAMPLE 2:

249.05 grams (0.882 moles) of oleic acid and 60.35 grams of toluene where charged in a 1 liter reaction flask equipped with Dean-Stark trap. Under nitrogen, the mixture was stirred and heated to 100°C. Over about 20 minutes, 128.77 grams (0.882 moles) of 3-(2-(dimethylamino)ethoxy) propylamine (DMAEPA) was added. The temperature was increased to about 165°C and held for 4 hours while removing water. Toluene was removed under vacuum. IR spectroscopy of the product confirmed formation of oleyl amide.

7.50 grams (0.0183 moles) of oleyl amide and 2.80 grams (0.0184 moles) of methyl salicylate were charged in a thick walled glass tube and sealed. The mixture was heated under nitrogen to 140°C and held for 12 hours. ¹H NMR spectroscopy of the product confirmed formation of the quaternary ammonium salt.

### EXAMPLE 3

preparatory PIBSI was prepared as follows: 207.75 grams (0.218 equivalents of anhydride) of PIBSA (made with about 1000 MW PIB and maleic anhydride) and 67.96 grams of toluene were charged in a 1 liter reaction flask equipped with Dean-Stark trap. Under nitrogen, the mixture was stirred and heated to 100°C. Over about 15 minutes, 30.24 grams (0.207 moles) of 3-(2-(dimethylamino)ethoxy)propylamine (DMAEPA) was added. The temperature was increased to about 160°C and held for 3 hours while removing water. Toluene was removed under vacuum. IR spectroscopy of the product confirmed formation of the succinimide.

67.20 grams (0.057 moles) of the above-obtained PIBSI and 8.69 grams (0.057 moles) of methyl salicylate were charged in a 250 ml reaction flask. The mixture was heated under nitrogen to 140°C and held for 6 hours. ¹H NMR spectroscopy of the product confirmed formation of the quaternary ammonium salt.

### EXAMPLE 4

In this Example, the ability of a gasoline-like fuel including the cavitation inhibitor of the present disclosure to reduce and/or minimize cavitation-induced damage to fuel system components will be evaluated. In the Experiment, the cavitation inhibitor will be oleyl dimethylaminopropylamine betaine and the gasoline-like fuel will be commercially available RON60 or RON91 gasoline having the properties of Table 2 below.

**Table 2**

| **Property** | **Units** | **RON 60 Gasoline** |
|---|---|---|
| Initial Boiling Point | °C | 41 |
| 10% Evaporation temperature | °C | 72 |
| 50% Evaporation temperature | °C | 99 |
| 90% evaporation temperature | °C | 124 |
| Final boiling point | °C | 134 |
| Vapor pressure | kPa | 45.0 |
| Density (15.56 C) | g/ml | 0.714 |
| Kinematic Viscosity | cSt | 0.593 |
| Wear Scar Diameter | Um | 240 |
| Aromatics | Volume % | 7.2 |
| Olefins | Volume % | 0.7 |
| Saturates | Volume % | 92.1 |
| Sulfur | Ppmw | 16.5 |
| H/C ratio | Mol/mol | 2.151 |
| Cetane Number (CN) | - | 34.4 |
| RON | | 56.6 |
| MON | | 54.8 |
| AKI | | 55.7 |
| Lower Heating value | MJ/kg | 44.018 |

The treat rate of the inhibitor in the fuel will be about 60 ppmw (58 ppmv). The fuel will be run through an instrumented bench fuel system apparatus including a Cummins design XPI common rail injection system capable of achieving fuel pressures up to about 2500 bar. This fuel system is found on at least 2014 Cummins ISX15 diesel engines (6 cylinder, 15 liters) and is a common example of on-road, heavy-duty engines. The evaluation will consistent of the 10 hour NATA durability cycle as shown in Table 3 below. This cycle will be repeated 40 times for a total of 400 hours. Further details of the test protocol and data analysis can be found in Tzanetakis et al., "Durability Study of high Pressure Common Rail Fuel Injection System Using Lubricity Additive Dosed Gasoline-Like Fuel," SAE Technical Paper 2018-01-0270, 2018, doi: 10.4271/2018-01-0270, which is incorporated herein in its entirety by reference.

**Table 3: NATO Durability Cycle Operating Points.**

| **Time (h)** | **NATO Speed/Load Pt.¹** | **Pump Speed (rpm)** | **SET Load Point²** | **Injection Duration (ms)³** | **Rail P (bar)** | **Fuel Rate (kg/h)⁴** |
|---|---|---|---|---|---|---|
| 0.5 | IDLE/0 | 600 | IDLE | 0.44 | 700 | 0.87 |
| 2.0 | 100/100 | 1800 | C100 | 1.82 | 2500 | 75.1 |
| 0.5 | GOV/0 | 2130 | IDLE | 0.44 | 700 | 0.87 |
| 1.0 | 75/100 | 1350 | B100 | 2.21 | 2200 | 64.3 |
| 2.0 | IDLE-100/0-100⁵ | 600-1800 | IDLE-C100 | 0.44-1.82 | 700-2500 | 0.87-76.6 |
| 0.5 | 60/100 | 1080 | A100 | 2.50 | 1850 | 53.0 |
| 0.5 | IDLE | 600 | IDLE | 0.44 | 700 | 0.87 |
| 0.5 | GOV | 1900 | C75 | 1.64 | 1975 | 63.6 |
| 2.0 | @MAX T/100 | 1100 | A100 | 2.50 | 1850 | 53.0 |
| 0.5 | 60/50 | 1080 | A25 | 0.98 | 1400 | 15.6 |

| | | | | | | |
|---|---|---|---|---|---|---|
| (1) Speed points specified in terms of % rated or as otherwise defined by the idling speed (IDLE), the governed speed (GOV), the speed at maximum engine torque (@MAX T), and load points specified in terms of % rated or equivalent accelerator pedal position, (2) accelerator pedal positions from steady-state SET operating points on the engine were used to define the injection duration, rail pressure, and fueling rate that most closely matched each NATO cycle load point definition, (3) Electronic signal duration, (4) Total fuel to all six injectors, (5) Duration at IDLE speed and 0% rated load is 4 min, duration at 100% rated speed and 100% rated load is 6min. | | | | | | |

Evaluations will include review of initial and final fuel system performance characteristics at various points within the NATO cycle, such as the fifth cycle (40 to 50 hour) as compared to the 40^{th} cycle (390 to 400 hour). It is anticipated that the fuel will not show any significant difference between initial and final fuel system performance. Analysis will include review the electric driving motor, torque flange, high pressure piston pump, rail pressure sensor for rail pressure, flexible controller module, fuel tank pressure, fuel feed temperature, gear pump inlet pressure, gear pump outlet pressure, high pressure pump inlet temperature, high pressure pump inlet pressure, common rail pressure, injector leakage flow pressure, injector leakage temperature, backflow temperature, backflow mass flow rate, injected fuel temperature, injected fuel mass flow rate, backflow temperature, and/or backflow pressure. It is expected that the driving power requirements will remain similar throughout the evaluation and the hardware will be able to achieve the desired rail pressure and fueling rates without significant adjustment by the controller.

Additionally, the fuel and lubricant oil will be analyzed throughout the evaluation. First, wear scar of the recirculated fuel every 50 hours will be performed. It will be expected that wear scar will remain consistent throughout the testing with an anticipated WSD between 150 and 275 microns when measured at 25°C. Metal content of the fuel exchanged every 50 hours will also be measured. Additionally, fuel and lubricant oil will be analyzed. It is expected that the fuel and lubricant metal content will be consistent to that or less than that reported in the Tzanetakis SAE paper discussed above.

Next, Injection rate analysis will be completed for the gasoline-like fuel with different injectors through the evaluation. It is expected that the additives herein will reduce or inhibit cavitation damage to the injectors and fuel pumps; thus, it is further expected that there will be little to no significant change to injection rates and other fuel injection parameters throughout the testing.

Lastly, at the conclusion of the testing, a hardware teardown analysis will be conducted of components in at least the high pressure pump head. FIGS. 1, 2, and 3 illustrate the high pressure pumping chamber dome with inlet and outlet portions, the high pressure pump inlet check valve plunger, and the high pressure pump inlet check valve stop that all show pitting damage believed to result from fuel cavitation when fuel *without* the cavitation inhibitor herein is used. It is expected that the after running the testing herein using the cavitation inhibitor, the damage to such parts will be visibly improved or eliminated.

It is to be understood that while the cavitation inhibitor, other fuel additives, compositions, and methods of this disclosure have been described in conjunction with the detailed description thereof and summary herein, the foregoing description is intended to illustrate and not limit the scope of the disclosure, which is defined by the scope of the appended claims. Other aspects, advantages, and modifications are within the scope of the claims. It is intended that the specification and examples be considered as exemplary only, with a true scope of the disclosure being indicated by the following claims.

Other embodiments of the present disclosure will be apparent to those skilled in the art from consideration of the specification and practice of the embodiments disclosed herein. As used throughout the specification and claims, "a" and/or "an" may refer to one or more than one. Unless otherwise indicated, all numbers expressing quantities of ingredients, properties such as molecular weight, percent, ratio, reaction conditions, and so forth used in the specification are to be understood as being modified in all instances by the term "about," whether or not the term "about" is present. Accordingly, unless indicated to the contrary, the numerical parameters set forth in the specification are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

It is to be understood that each component, compound, substituent or parameter disclosed herein is to be interpreted as being disclosed for use alone or in combination with one or more of each and every other component, compound, substituent or parameter disclosed herein.

It is further understood that each range disclosed herein is to be interpreted as a disclosure of each specific value within the disclosed range that has the same number of significant digits. Thus, for example, a range from 1 to 4 is to be interpreted as an express disclosure of the values 1, 2, 3 and 4 as well as any range of such values. It is also further understood that any range between the endpoint values within a described range is also discussed herein. Thus, a range from 1 to 4 also means a range from 1 to 3, 1 to 2, 2 to 4, 2 to 3, and so forth.

It is further understood that each lower limit of each range disclosed herein is to be interpreted as disclosed in combination with each upper limit of each range and each specific value within each range disclosed herein for the same component, compounds, substituent or parameter. Thus, this disclosure to be interpreted as a disclosure of all ranges derived by combining each lower limit of each range with each upper limit of each range or with each specific value within each range, or by combining each upper limit of each range with each specific value within each range.

Furthermore, specific amounts/values of a component, compound, substituent or parameter disclosed in the description or an example is to be interpreted as a disclosure of either a lower or an upper limit of a range and thus can be combined with any other lower or upper limit of a range or specific amount/value for the same component, compound, substituent or parameter disclosed elsewhere in the application to form a range for that component, compound, substituent or parameter.

**The invention further relates to the following numbered embodiments:**
1. A method of reducing cavitation damage in a common-rail injection engine, the method comprising:
   providing a gasoline-like fuel composition at a pressure of about 350 to about 5,000 bar to a fuel injector and/or a high pressure pumping system of a common-rail injection engine and combusting the fuel composition in the engine;
   the gasoline-like fuel composition including a major amount of gasoline-like fuel and a minor amount of a cavitation additive including a quaternary ammonium compound.
2. The method of reducing cavitation damage in a common-rail injection engine according to embodiment 1, wherein the quaternary ammonium compound has a structure of Formula I; 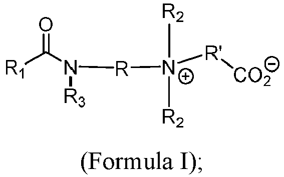 wherein
   R and R' are independently alkylene linkers having 1 to 10 carbon atoms;
   R₁ is a hydrocarbyl group or optionally substituted hydrocarbyl group, or an aryl group or optionally substituted aryl group;
   R₂ is independently a linear or branched C1 to C4 alkyl group; and
   R₃ is hydrogen or a C1 to C4 alkyl group.
3. The method of reducing cavitation damage in a common-rail injection engine according to embodiment 1 or 2, wherein the fuel composition includes about 10 to about 1000 ppmw of the cavitation additive.
4. The method of reducing cavitation damage in a common-rail injection engine according to any previous embodiment, wherein the engine is a common-rail ignition diesel engine.
5. The method of reducing cavitation damage in a common-rail injection engine according to any preceding embodiment, wherein R and R' are independently alkylene linkers having 1 to 3 carbon atoms and R₁ is a C8 to C20 hydrocarbyl group.
6. The method of reducing cavitation damage in a common-rail injection engine according to any preceding embodiment, wherein R' includes a methylene linker.
7. The method of reducing cavitation damage in a common-rail injection engine according to any preceding embodiment, wherein R₂ is a methyl group.
8. The method of reducing cavitation damage in a common-rail injection engine according to any preceding embodiment, wherein the reduction in cavitation damage occurs in one or more of an inlet cavity to a fuel pumping chamber, a fuel inlet check valve plunger, a fuel inlet check valve stop, or any combination thereof.
9. The method of reducing cavitation damage in a common-rail injection engine according to any preceding embodiment, wherein the quaternary ammonium salt is formed by the reaction of an alkyl carboxylate with a compound obtained by reacting a hydrocarbyl substituted acylating agent and an amine, wherein the amine has the structure 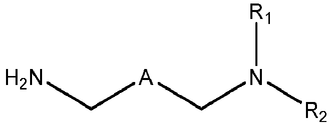 wherein
   A is a hydrocarbyl linker with 2 to 10 carbon units and including one or more carbon units thereof independently replaced with a bivalent moiety selected from the group consisting of -O-, -N(R')-, -C(O)-, -C(O)O-, -C(O)NR';
   R₁ and R₂ are independently alkyl groups containing 1 to 8 carbon atoms; and
   R' is independently a hydrogen or a group selected from C₁₋₆ aliphatic, phenyl, or alkylphenyl.
10. The method of reducing cavitation damage in a common-rail injection engine according to embodiment 9, wherein the alkyl carboxylate is alkyl oxalate, alkyl salicylate, or a combination thereof.
11. The method of reducing cavitation damage in a common-rail injection engine according to embodiment 9, wherein the alkyl group in the alkyl carboxylate is C1 to C6 alkyl.
12. The method of reducing cavitation damage in a common-rail injection engine according to embodiment 9, wherein A is -(CH₂)ᵣ-[X-(CH₂)_{r'}]ₚ- with each of r, r', and p independently being 1, 2, 3, or 4 and X being O or NR" with R" being hydrogen or a hydrocarbyl group.
13. The method of reducing cavitation damage in a common-rail injection engine according to embodiment 9, wherein X is oxygen.
14. The method of reducing cavitation damage in a common-rail injection engine according to embodiment 9, wherein the amine is selected from 3-(2-(dimethyl amino)ethoxy)propylamine, N,N-dimethyldipropylenetriamine, and mixtures thereof.
15. The method of reducing cavitation damage in a common-rail injection engine according to embodiment 9, wherein the hydrocarbyl substitued acylating agent is selected from a hydrocarbyl substituted dicarboxylic acid or anhydride derivative thereof, a fatty acid, or mixtures thereof.
16. The method of reducing cavitation damage in a common-rail injection engine according to embodiment 9, wherein the hydrocarbyl substituent has a number average molecular weight of about 200 to about 2500 as measured by GPC using polystyrene as a calibration reference.
17. The method of reducing cavitation damage in a common-rail injection engine according to embodiment 9, wherein the formed quaternary ammonium salt has the structure 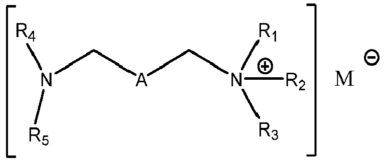 wherein
   A is a hydrocarbyl linker with 2 to 10 carbon units and including one or more carbon units thereof independently replaced with a bivalent moiety selected from the group consisting of -O-, -N(R')-, -C(O)-, -C(O)O-, -C(O)NR';
   R₁, R₂, and R₃ are independently alkyl groups containing 1 to 8 carbon atoms; and R' is independently a hydrogen or a group selected from C₁₋₆ aliphatic, phenyl, or alkylphenyl; and
   R₄ and R₅ are independently selected from a hydrogen, an acyl group, or a hydrocarbyl substituted acyl group, wherein if one of R₄ or R₅ is hydrogen, then the other of R₄ and R₅ is the acyl group or the hydrocarbyl substituted acyl group, if both R₄ and R₅ include carbonyl moieties, then one of R₄ and R₅ includes the acyl group and the other of R₄ and R₅ includes the hydrocarbyl substitued acyl group, and R₄ and R₅ together with the N atom to which they are attached, combine to form a ring moiety; and
   M⁻ is a carboxylate.
18. The method of reducing cavitation damage in a common-rail injection engine according to any preceding embodiment, where the fuel is gasoline.

## Claims

1. A use of a cavitation additive including a quaternary ammonium compound in a gasoline-like fuel composition for reducing cavitation damage in a common-rail injection engine.

2. The use of claim 1, comprising:
providing a gasoline-like fuel composition at a pressure of at least 350 bar, preferably at greater than 350 bar or at 350 to 5,000 bar, to a fuel injector and/or a high pressure pumping system of a common-rail injection engine and combusting the fuel composition in the engine;
wherein the gasoline-like fuel composition includes a major amount of gasoline-like fuel and a minor amount of the cavitation additive including the quaternary ammonium compound.

3. The use according to claim 1 or 2, wherein the quaternary ammonium compound has a structure of Formula I; 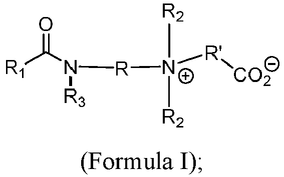 wherein
R and R' are independently alkylene linkers having 1 to 10 carbon atoms;
R₁ is a hydrocarbyl group or optionally substituted hydrocarbyl group, or an aryl group or optionally substituted aryl group;
R₂ is independently a linear or branched C1 to C4 alkyl group; and
R₃ is hydrogen or a C1 to C4 alkyl group.

4. The use according to claim 3, wherein R and R' are independently alkylene linkers having 1 to 3 carbon atoms and R₁ is a C8 to C20 hydrocarbyl group; and/or
wherein R' includes a methylene linker; and/or
wherein R₂ is a methyl group.

5. The use according to claim 1 or 2, wherein the quaternary ammonium salt is formed by the reaction of an alkyl carboxylate with a compound obtained by reacting a hydrocarbyl substituted acylating agent and an amine, wherein the amine has the structure 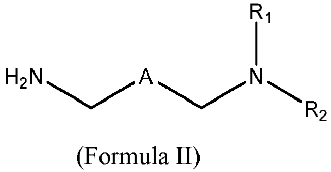 wherein
A is a hydrocarbyl linker with 2 to 10 carbon units and including one or more carbon units thereof independently replaced with a bivalent moiety selected from the group consisting of -O-, -N(R')-, -C(O)-, -C(O)O-, -C(O)NR';
R₁ and R₂ are independently alkyl groups containing 1 to 8 carbon atoms; and
R' is independently a hydrogen or a group selected from C₁₋₆ aliphatic, phenyl, or alkylphenyl.

6. The use according to claim 5, wherein the alkyl carboxylate is alkyl oxalate, alkyl salicylate, or a combination thereof; and/or
wherein the alkyl group in the alkyl carboxylate is C1 to C6 alkyl; and/or
wherein A is -(CH₂)ᵣ-[X-(CH₂)_{r'}]ₚ- with each of r, r', and p independently being 1, 2, 3, or
4 and X being O or NR" with R" being hydrogen or a hydrocarbyl group.

7. The use according to claim 5 or 6, wherein X is oxygen.

8. The use according to any one of claims 5 to 7, wherein the amine is selected from 3-(2-(dimethyl amino)ethoxy)propylamine, N,N-dimethyldipropylenetriamine, and mixtures thereof.

9. The use according to any one of claims 5 to 8, wherein the hydrocarbyl substituted acylating agent is selected from a hydrocarbyl substituted dicarboxylic acid or anhydride derivative thereof, a fatty acid, or mixtures thereof.

10. The use according to any one of claims 5 to 9, wherein the hydrocarbyl substituent has a number average molecular weight of 200 to 2500 as measured by GPC using polystyrene as a calibration reference.

11. The use according to any one of claims 5 to 10, wherein the formed quaternary ammonium salt has the structure 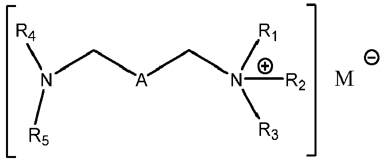 wherein
A is a hydrocarbyl linker with 2 to 10 carbon units and including one or more carbon units thereof independently replaced with a bivalent moiety selected from the group consisting of -O-, -N(R')-, -C(O)-, -C(O)O-, -C(O)NR';
R₁, R₂, and R₃ are independently alkyl groups containing 1 to 8 carbon atoms; and
R' is independently a hydrogen or a group selected from C₁₋₆ aliphatic, phenyl, or alkylphenyl; and
R₄ and R₅ are independently selected from a hydrogen, an acyl group, or a hydrocarbyl substituted acyl group, wherein if one of R₄ or R₅ is hydrogen, then the other of R₄ and R₅ is the acyl group or the hydrocarbyl substituted acyl group, if both R₄ and R₅ include carbonyl moieties, then one of R₄ and R₅ includes the acyl group and the other of R₄ and R₅ includes the hydrocarbyl substituted acyl group, and R₄ and R₅ together with the N atom to which they are attached, combine to form a ring moiety; and
M⁻ is a carboxylate.

12. The according to any one of claims 1 to 11, wherein the fuel is gasoline.

13. The use according to any one of claims 1 to 12, wherein the fuel composition includes 10 to 1000 ppmw of the cavitation additive.

14. The use according to any one of claims 1 to 13, wherein the engine is a common-rail ignition diesel engine.

15. The use according to any one of claims 1 to 14, wherein the reduction in cavitation damage occurs in one or more of an inlet cavity to a fuel pumping chamber, a fuel inlet check valve plunger, a fuel inlet check valve stop, or any combination thereof.
